# EUROPEAN PATENT APPLICATION

(11) **EP 4 763 229 A1**
(43) Date of publication of application: **24.06.2026**
(21) Application number: 25225464.4
(22) Date of filing: 19.12.2025
(51) Int. Cl.: A61K 47/68, A61P 35/00, C07K 16/30

(54) **ANTI-GPC3 ANTIBODY DRUG CONJUGATES**

(30) Priority: 19.12.2024 US 202463736298 P
(71) Applicant: AbbVie Inc., North Chicago, IL 60064 (US)
(72) Inventor: FAIVRE, Emily Jean, North Chicago, IL 60064 (US); O'HAINMHIRE, Eoghainin, North Chicago, IL 60064 (US)
(74) Representative: J A Kemp LLP

(57) **Abstract**

The present disclosure provides GPC3 antibody drug conjugates (ADCs), including compositions and methods of using such ADC.

## Description

### 1. SEQUENCE LISTING

The instant application contains a Sequence Listing which has been submitted electronically in XML format and is hereby incorporated by reference in its entirety. Said XML copy, created on.

### 2. TECHNICAL FIELD

The present application pertains to anti-GPC3 antibody drug conjugates (ADCs).

### 3. BACKGROUND

GPC3 is a hepatocellular carcinoma (HCC) tumor antigen with high expression in the majority of tumors (Yamauchi et al, 2005. The glypican 3 oncofetal protein is a promising diagnostic marker for hepatocellular carcinoma. Mod Pathol 18, 1591-1598). Expression of GPC3 is associated with poor survival and is maintained in the post-treatment setting and independent of viral status or race/ethnicity (Guo et al, 2020. Glypican-3: A New Target for Diagnosis and Treatment of Hepatocellular Carcinoma. J Cancer. 2020 Feb 3;11(8):2008-2021. doi: 10.7150/jca.39972). As a member of the glypican family of heparan sulfate proteoglycans, GPC3 is tethered to the cell membrane via a glycosylphosphatidylinositol (GPI) anchor and internalizes upon antibody binding. GPC3 is expressed during development where it functions to modify WNT and hedgehog signaling. However, GPC3 protein is not expressed in most normal adult tissues (Guo et al, 2020. Glypican-3: A New Target for Diagnosis and Treatment of Hepatocellular Carcinoma. J Cancer. 2020 Feb 3;11(8):2008-2021. doi: 10.7150/jca.39972).

Conventional therapeutic treatments for cancer are often ineffective, thus there remains a need to develop more targeted and potent therapies for treating HCC. An antibody drug conjugate (ADC) comprising an antibody targeting GPC3 conjugated to a cytotoxic drug via a chemical linker provides a targeted therapy for treating patients with HCC tumors that express GPC3.

### 4. SUMMARY

Provided herein is an anti-GPC3 antibody drug conjugate of formula (IV): 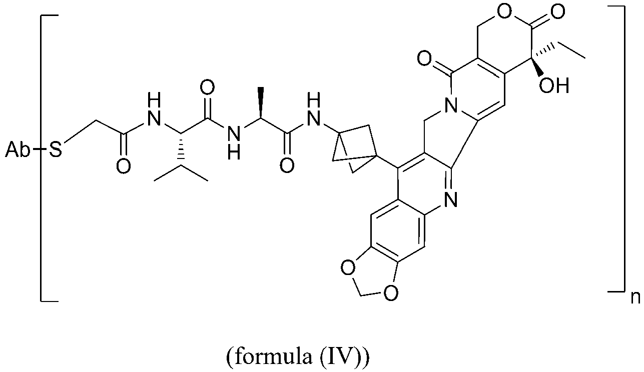
wherein n is an integer from 1 to 6, and wherein Ab is an anti-GPC3 antibody comprising a heavy chain variable region comprising a CDR-H1, a CDR-H2, and a CDR-H3; and a light chain variable region comprising a CDR-L1, a CDR-L2, and a CDR-L3, wherein
CDR-H1 has the amino acid sequence shown as SEQ ID NO: 3,
CDR-H2 has the amino acid sequence shown as SEQ ID NO: 4,
CDR-H3 has the amino acid sequence shown as SEQ ID NO: 5;
CDR-L1 has the amino acid sequence shown as SEQ ID NO: 6,
CDR-L2 has the amino acid sequence shown as SEQ ID NO: 7, and
CDR-L3 has the amino acid sequence shown as SEQ ID NO: 8.

In some embodiments, the anti-GPC3 antibody of the antibody drug conjugate comprises (a) a heavy chain variable region having the amino acid sequence shown as SEQ ID NO: 1 and (b) a light chain variable region having the amino acid sequence shown as SEQ ID NO: 2.

In some embodiments, the anti-GPC3 antibody of the antibody drug conjugate is an IgG1 antibody.

In some embodiments, the anti-GPC3 antibody of the antibody drug conjugate comprises (a) a heavy chain having the amino acid shown as SEQ ID NO: 9 and (b) a light chain having the amino acid sequence shown as SEQ ID NO: 10.

In some embodiments, the anti-GPC3 antibody drug conjugate has the structure of formula (IV): 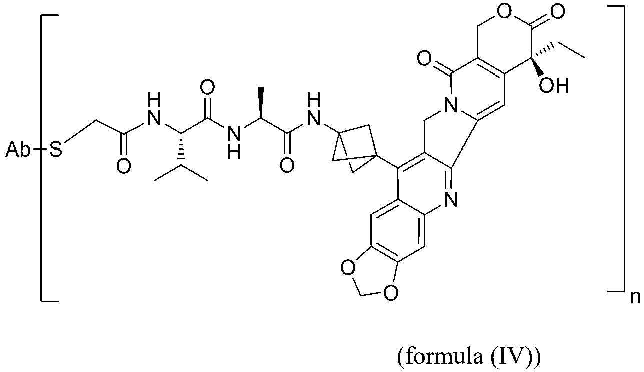 wherein n is an integer from 1 to 6, and wherein Ab is an anti-GPC3 antibody comprising: two heavy chains each having an amino acid sequence shown as SEQ ID NO: 9 and two light chains each having an amino acid sequence shown as SEQ ID NO: 10.

In some embodiments, the anti-GPC3 antibody drug conjugate has n of 2.

In some embodiments, the anti-GPC3 antibody drug conjugate has n of 4.

In some embodiments, the anti-GPC3 antibody drug conjugate has n of 6.

In some embodiments, the anti-GPC3 antibody drug conjugate has n of 1.

Also provided herein is a composition comprising a therapeutically effective amount of an anti-GPC3 antibody drug conjugate disclosed herein, wherein the predominant species of the antibody drug conjugate in the composition has an n of 2.

Also provided herein is a pharmaceutical composition comprising a therapeutically effective amount of an anti-GPC3 antibody drug conjugate disclosed herein and a pharmaceutically acceptable excipient, wherein the composition has a DAR of 2 or about 2.

Also provided herein is a method of treating hepatocellular carcinoma (HCC), wherein the method comprises administering a therapeutically effective amount of an anti-GPC3 antibody drug conjugate disclosed herein to a patient in need thereof.

Also provided herein is a method of treating HCC, wherein the method of administering a therapeutically effective amount of a pharmaceutical composition disclosed herein comprising a therapeutically effective amount of an anti-GPC3 antibody drug conjugate disclosed herein to a patient in need thereof.

Also provided are nucleic acids encoding an antibody comprising a heavy chain sequence of SEQ ID NO: 9 or SEQ ID NO: 11, and a light chain sequence of SEQ ID NO: 10.

### 5. BRIEF DESCRIPTION OF THE DRAWINGS

**FIG. 1** shows comparative data between mAb1 and GC33 ADCs. FIG. 1A shows growth inhibition of HepG2, Hep3B, and SK-HEP1-GPC3 cells in 2D culture following administration of DAR = 2 (top) and DAR = 6 (bottom) versions of mAb1 and GC33. FIG. 1B shows growth inhibition by ADC-1 and DAR2 GC33 in HepG2, Hep3B, and SK-HEP1-GPC3 cells in 3D spheroids.

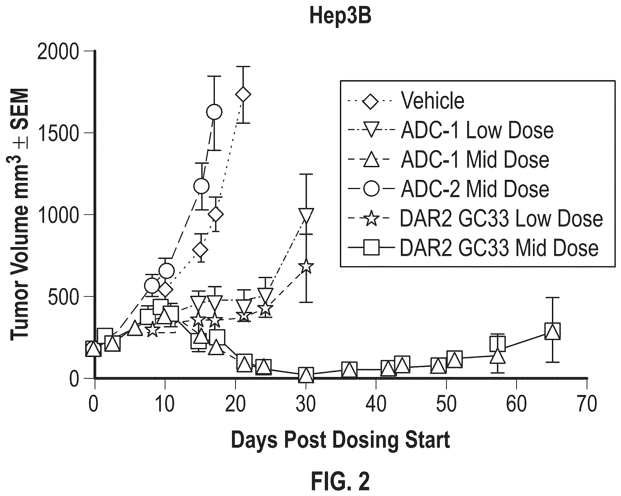

**FIG. 2** shows dose response of ADC-1 and DAR2 GC33 ADC treatment in a human HCC Hep3B CDX mouse model.
**FIG. 3** shows dose response of ADC-1 treatment in a human HCC HepG2 CDX mouse model.
**FIG. 4** shows dose response of ADC-1 treatment in a human HCC LI1068 patient-derived xenograft (PDX) mouse model.
**FIG. 5** shows dose response of ADC-1 treatment in a human HCC LI6610 PDX mouse model.

### 6. DETAILED DESCRIPTION

As used in this disclosure, the singular forms "a", "an" and "the" include plural referents unless the context clearly dictates otherwise. The terms "a" (or "an"), as well as the terms "one or more," and "at least one" can be used interchangeably herein unless the context clearly dictates otherwise.

As used in this disclosure and unless otherwise specified, the terms "about" and "approximately" generally refer to a range of numbers that one of skill in the art would consider equivalent to the recited value (i.e., having the same function or result). In many instances, the terms "about" and "approximately" may include numbers that are rounded to the nearest significant figure. In specific embodiments, the terms "about" and "approximately" shall be construed so as to allow normal variation as judged by a person of ordinary skill in the art, such as, for example, a variation within 20% or 10% or 5%. In specific embodiments, the terms "about" and "approximately" encompass the exact value recited. Unless the context clearly dictates otherwise, all numerical values provided herein are modified by the term about.

In certain embodiments, disclosed herein are anti-GPC3 ADCs comprising TOP1 inhibitors, and methods of using the ADCs.

### 6.1. Topoisomerase 1 Inhibitors (TOPli)

Topoisomerase 1 (TOP1) removes supercoils formed during DNA replication. TOP1 inhibitors (TOP1i) can bind and stabilize TOP1-DNA complexes, inducing DNA strand breakage and apoptosis.

In certain embodiments, provided herein is a topoisomerase I inhibitor drug ("TOP1i drug") according to structural formula (I), which may be purposed for targeted delivery to cells by conjugation to an anti-GPC3 antibody.

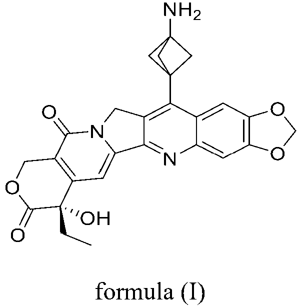

In certain embodiments, the TOP1i drug is (7*S*)-14-(3-aminobicyclo[1.1.1]pentan-1-yl)-7-ethyl-7-hydroxy-2*H*,10*H*-[1,3]dioxolo[4,5-*g*]pyrano[3',4':6,7]indolizino[1,2-*b*]quinoline-8,11(7*H*,13*H*)-dione.

In certain embodiments, the TOP1i drug as contemplated herein may be conjugated to an antibody via a linker as shown in structural formula (II): 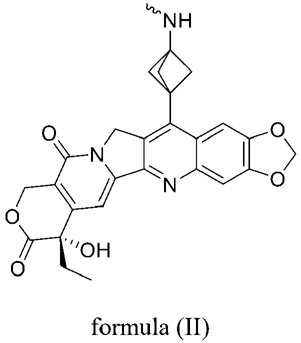 wherein 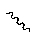 represents the point of attachment of a linker to the TOP1i drug.

### 6.1.1. Linker-Drug LD1 (Structural Formula (III))

In certain embodiments, a TOP1i linker drug (LD1) is a compound according to formula (III). In certain embodiments, LD1 is (2*S*)-2-(2-bromoacetamido)-*N*-[(2*S*)-1-({3-[(7*S*)-7-ethyl-7-hydroxy-8,11-dioxo-7,8,11,13-tetrahydro-2*H*,10*H*-[1,3]dioxolo[4,5-*g*]pyrano[3',4':6,7]indolizino[1,2-*b*]quinolin-14-yl]bicyclo[1.1.1]pentan-1-yl}amino)-1-oxopropan-2-yl]-3-methylbutanamide.

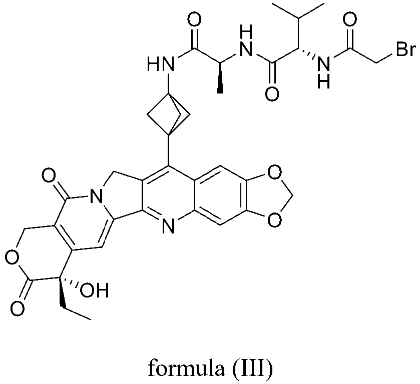

### 6.2. ANTI-GPC3 ADCs

In certain embodiments, the TOP1i drug as described herein (i.e., formula (I)) may be conjugated to an anti-GPC3 antibody to form an anti-GPC3 TOP1i antibody drug conjugate. In certain embodiments, the present disclosure provides anti-GPC3 TOP1i ADC for therapeutic use in the treatment of GPC3 expressing HCC tumors. In certain embodiments the anti-GPC3 antibody is mAb1, which was surprisingly well tolerated as a TOP1i ADC in non-human primates in contrast to a TOP1i ADC using the GC33 (codrituzumab) anti-GPC3 antibody (Zhu, et al. 2013. First-in-man phase I study of GC33, a novel recombinant humanized antibody Against Glypican-3, in patients with advanced hepatocellular carcinoma. Clin. Cancer Res. DOI: 10.1158/1078-0432.CCR-12-2616).

In certain embodiments, TOP1i drugs are conjugated to the anti-GPC3 antibody by way of a linker moiety. In certain embodiments, the linkers connect the TOP1i drug to the anti-GPC3 antibody by forming a covalent linkage to the TOP1i drug at one location and a covalent linkage to the antibody at another. In certain embodiments, the covalent linkages are formed by reaction between functional groups on the linker and functional groups on the TOP1i drug and the anti-GPC3 antibody. In certain embodiments, the anti-GPC3 ADC of the present disclosure is comprised of an anti-GPC3 antibody conjugated to the TOP1i linker drug of formula (III).

In certain embodiments, "Ab" refers to an anti-GPC3 antibody comprising a heavy chain having an amino acid sequence shown as SEQ ID NO: 9 and a light chain having an amino acid sequence shown as SEQ ID NO: 10. In certain embodiments, Ab is conjugated to the TOP1i linker-drug of formula (III).

### 6.2.1. Ab ANTI-GPC3 Antibody

In certain embodiments, Ab is a fully human anti-GPC3 IgG1 monoclonal antibody. In certain embodiments, Ab comprises variable regions and CDRs (complementary determining regions) identified according to rules developed in the art and/or by aligning sequences against a database of known variable regions.

Methods for identifying these regions are described in Kontermann and Dubel, eds., Antibody Engineering, Springer, New York, N.Y., 2001 and Dinarello et al., Current Protocols in Immunology, John Wiley and Sons Inc., Hoboken, N.J., 2000. For example, CDRs may be identified in accordance with one of the schemes provided by Kabat et al. (1991) Sequences of Proteins of Immunological Interest (5th Ed.), U.S. Dept. of Health and Human Services, PHS, NIH, NIH Publication No. 91-3242 (referred to herein as "Kabat"); or in accordance with AbM (Oxford Molecular/MSI Pharmacopia) (referred to herein as "AbM"). AbM can be obtained from the Abysis database at www.bioinf.org.uk/abs (maintained by A.C. Martin in the Department of Biochemistry & Molecular Biology University College London).

In certain embodiments, Ab comprises a heavy chain having an amino acid sequence shown as SEQ ID NO: 9 (variable region is **bold** and has an amino acid sequence shown as SEQ ID NO: 1; constant region is *italicized*; CDRs are underlined and have amino acid sequences shown as SEQ ID NOs: 3, 4 and 5 respectively, in order of appearance):

EVQLVESGGGLVQPGGSLRLSCAASGFTFSGFGMNWVRQAPGKGLEWVSYTSS
SSRTIYYADSVKGRFTISRDNAQNSLYLQMNSLRDEDTAVYYCARDSSGWYDAF
DIWGQGTMVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK

(SEQ ID NO: 9).

In certain embodiments, Ab comprises a heavy chain having the C-terminal lysine truncated, e.g., an amino acid sequence shown as SEQ ID NO: 11 (variable region is **bold** and has an amino acid sequence shown as SEQ ID NO: 1; constant region is *italicized*; CDRs are underlined and have amino acid sequences shown as SEQ ID NOs: 3, 4 and 5 respectively, in order of appearance):

EVQLVESGGGLVQPGGSLRLSCAASGFTFSGFGMNWVRQAPGKGLEWVSYTSS
SSRTIYYADSVKGRFTISRDNAQNSLYLQMNSLRDEDTAVYYCARDSSGWYDAF
DIWGQGTMVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPG

(SEQ ID NO: 11).

In certain embodiments, Ab comprises a light chain having an amino acid sequence shown as SEQ ID NO: 10 (variable region is **bold** and has an amino acid sequence shown as SEQ ID NO: 2; constant region is *italicized*; CDRs are underlined and have amino acid sequences shown as SEQ ID NOs: 6, 7 and 8 respectively, in order of appearance):

DIQLTQSPSFLSASVGDRVTITCRASQDISSYLAWYQQEPGKAPKLLIYATSTLQS
GVPSRFSGSGSGTEFTLTITSLQPEDFATYYCQQLNSYPWTFGQGTKVEIKRTVA
APSVFIFPPSDEQLKSGTASVVCLLNNFYPREAKVQWKVDNALQSGNSQESVTEQDSKDS
TYSLSSTLTLSKADYEKHKVYACEVTHQGLSSPVTKSFNRGEA
(SEQ ID NO: 10).

In certain embodiments, Ab comprises two heavy chains each having an amino acid sequence shown as SEQ ID NO: 9 and two light chains each having an amino acid sequence shown as SEQ ID NO: 10.

In certain embodiments, Ab comprises two heavy chains each having an amino acid sequence shown as SEQ ID NO: 11 and two light chains each having an amino acid sequence shown as SEQ ID NO: 10.

In certain embodiments, Ab comprises a CDR-H1 having an amino acid sequence shown as SEQ ID NO: 3; a CDR-H2 having an amino acid sequence shown as SEQ ID NO: 4; a CDR-H3 having an amino acid sequence shown as SEQ ID NO 5; a CDR-L1 having an amino acid sequence shown as SEQ ID NO: 6; a CDR-L2 having an amino acid sequence shown as SEQ ID NO 7; and a CDR-L3 having an amino acid sequence shown as SEQ ID NO 8. The amino acid sequence of CDR-H1 was identified using the AbM definition, and the remaining CDR amino acid sequences were identified using the Kabat definition.

In certain embodiments, Ab comprises a heavy chain variable domain having an amino acid sequence shown as SEQ ID NO: 1 and a light chain variable domain having an amino acid sequence shown as SEQ ID NO: 2.

In certain embodiments, provided herein are one or more nucleic acids encoding an antibody comprising a heavy chain sequence of SEQ ID NO: 9 or SEQ ID NO: 11, and a light chain sequence of SEQ ID NO: 10. In other embodiments the invention comprises a vector comprising one or more of the above described nucleic acids or a host cell comprising said nucleic acids or vectors. Nucleic acids of the invention can be obtained using standard molecular biology techniques. For antibodies expressed by hybridomas (e.g., hybridomas prepared as described in the Examples below), cDNAs encoding the light and heavy chains of the antibody can be obtained by standard PCR amplification or cDNA cloning techniques.

In certain embodiments Ab, the anti-GPC3 antibody may be in a format, such as an IgG₁ or IgG₄ format, that has been modified to confer desired properties, such as having the Fc mutated to reduce or "silence" effector function or extend half-life. In embodiments where half-life extending and/or Fc silencing mutations are introduced, Ab may comprise the heavy chain sequences in the antibody combinations shown in Table 12. SEQUENCE LISTING TABLE for SEQ ID NOs: 12-117.

Embodiments of Ab, the anti-GPC3 antibody, may comprise an IgG₁ heavy chain comprising amino acid sequences according to SEQ ID NOs: 9, 11, and 12-45. In embodiments, Ab may comprise a wild type IgG₁ heavy chain comprising amino acid sequences according to SEQ ID NO: 9 (with terminal lysine) and SEQ ID NO: 11 (without terminal lysine). In embodiments, the IgG₁ heavy chain comprises Fc silencing mutations selected from L234A, L235A (LALA) or L234S, L235T, G236R (STR). In embodiments, Ab comprises an IgG₁ heavy chain comprising the mutations L234A, L235A (LALA) with or without terminal lysine. In embodiments, Ab comprises an IgG1 heavy chain comprising the mutations L234S, L235T, G236R (STR) with or without terminal lysine.

In embodiments, Ab, the anti-GPC3 antibody, may comprise a heavy chain IgG₁ comprising half-life extension mutations selected from M252Y, S254T, T256E (YTE); Q311R, M428E, N434W (REW); M428L, N434S (LS); L309D, Q311H, N434S (DHS); or T250Q, M428L (QL). In embodiments, Ab comprises an IgG1 heavy chain comprising M252Y, S254T, T256E (YTE) with or without terminal lysine. In embodiments, Ab comprises an IgG₁ heavy chain comprising Q311R, M428E, N434W (REW) with or without terminal lysine. In embodiments, Ab comprises an IgG₁ heavy chain comprising M428L, N434S (LS) with or without terminal lysine. In embodiments, Ab comprises an IgG₁ heavy chain comprising L309D, Q311H, N434S (DHS) with or without terminal lysine. In embodiments, Ab comprises an IgG₁ heavy chain comprising T250Q, M428L (QL) with or without terminal lysine.

In embodiments, Ab, the anti-GPC3 antibody, may comprise both Fc silencing mutations and half-life extension mutations. In embodiments, Ab comprises an IgG₁ heavy chain comprising the mutations L234A, L235A (LALA) and M252Y, S254T, T256E (YTE) with or without terminal lysine. In embodiments, Ab comprises an IgG₁ heavy chain comprising the mutations L234S, L235T, G236R (STR) and M252Y, S254T, T256E (YTE) with or without terminal lysine. In embodiments, Ab comprises an IgG1 heavy chain comprising the mutations L234A, L235A (LALA) and Q311R, M428E, N434W (REW) with or without terminal lysine. In embodiments, Ab comprises an IgG₁ heavy chain comprising the mutations L234S, L235T, G236R (STR) and Q311R, M428E, N434W (REW) with or without terminal lysine. In embodiments, Ab comprises an IgG1 heavy chain comprising the mutations L234A, L235A (LALA) and M428L, N434S (LS) with or without terminal lysine. In embodiments, Ab comprises an IgG₁ heavy chain comprising the mutations L234S, L235T, G236R (STR) and M428L, N434S (LS) with or without terminal lysine. In embodiments, Ab comprises an IgG₁ heavy chain comprising the mutations L234A, L235A (LALA) and L309D, Q311H, N434S (DHS) with or without terminal lysine. In embodiments, Ab comprises an IgG₁ heavy chain comprising the mutations L234S, L235T, G236R (STR) and L309D, Q311H, N434S (DHS) with or without terminal lysine. In embodiments, Ab comprises an IgG₁ heavy chain comprising the mutations L234A, L235A (LALA) and T250Q, M428L (QL) with or without terminal lysine. In embodiments, Ab comprises an IgG₁ heavy chain comprising the mutations L234S, L235T, G236R (STR) and T250Q, M428L (QL) with or without terminal lysine.

Embodiments of Ab, the anti-GPC3 antibody may comprise an IgG₄ heavy chain comprising amino acid sequences according to SEQ ID NOs: 46-117. In embodiments, Ab may comprise a wild type IgG₄ heavy chain comprising amino acid sequences according to SEQ ID NO: 82 (with terminal lysine) and SEQ ID NO: 83 (without terminal lysine). In embodiments, Ab may comprise the IgG₄ heavy chain further comprising an S228P mutation according to SEQ ID NO: 46 (with terminal lysine) and SEQ ID NO: 47 (without terminal lysine). In embodiments, the IgG₄ heavy chain comprises Fc silencing mutations selected from F234A, L235A (FALA) or L234S, L235T, G236R (STR). In embodiments, Ab comprises an IgG₄ heavy chain comprising the mutations F234A, L235A (FALA) with or without terminal lysine and with or without an S228P mutation. In embodiments, Ab comprises an IgG₄ heavy chain comprising the mutations L234S, L235T, G236R (STR) with or without terminal lysine and with or without an S228P mutation.

In embodiments, Ab, the anti-GPC3 antibody, may comprise an IgG₄ heavy chain comprising half-life extension mutations selected from M252Y, S254T, T256E (YTE); Q311R, M428E, N434W (REW); M428L, N434S (LS); L309D, Q311H, N434S (DHS); or T250Q, M428L (QL). In embodiments, Ab comprises an IgG₄ heavy chain comprising M252Y, S254T, T256E (YTE) with or without terminal lysine and with or without an S228P mutation. In embodiments, Ab comprises an IgG₄ heavy chain comprising Q311R, M428E, N434W (REW) with or without terminal lysine and with or without an S228P mutation. In embodiments, Ab comprises an IgG₄ heavy chain comprising M428L, N434S (LS) with or without terminal lysine and with or without an S228P mutation. In embodiments, Ab comprises an IgG₄ heavy chain comprising L309D, Q311H, N434S (DHS) with or without terminal lysine and with or without an S228P mutation. In embodiments, Ab comprises an IgG₄ heavy chain comprising T250Q, M428L (QL) with or without terminal lysine and with or without an S228P mutation. In embodiments, Ab may comprise both Fc silencing mutations and half-life extension mutations.

In embodiments, Ab, the anti-GPC3 antibody, comprises an IgG₄ heavy chain comprising the mutations F234A, L235A (FALA) and M252Y, S254T, T256E (YTE) with or without terminal lysine and with or without an S228P mutation. In embodiments, Ab comprises an IgG₄ heavy chain comprising the mutations L234S, L235T, G236R (STR) and M252Y, S254T, T256E (YTE) with or without terminal lysine and with or without an S228P mutation. In embodiments, Ab comprises an IgG₄ heavy chain comprising the mutations F234A, L235A (FALA) and Q311R, M428E, N434W (REW) with or without terminal lysine and with or without an S228P mutation. In embodiments, Ab comprises an IgG₄ heavy chain comprising the mutations L234S, L235T, G236R (STR) and Q311R, M428E, N434W (REW) with or without terminal lysine and with or without an S228P mutation. In embodiments, Ab comprises an IgG₄ heavy chain comprising the mutations F234A, L235A (FALA) and M428L, N434S (LS) with or without terminal lysine and with or without an S228P mutation. In embodiments, Ab comprises an IgG₄ heavy chain comprising the mutations L234S, L235T, G236R (STR) and M428L, N434S (LS) with or without terminal lysine and with or without an S228P mutation. In embodiments, Ab comprises an IgG₄ heavy chain comprising the mutations F234A, L235A (FALA) and L309D, Q311H, N434S (DHS) with or without terminal lysine and with or without an S228P mutation. In embodiments, Ab comprises an IgG₄ heavy chain comprising the mutations L234S, L235T, G236R (STR) and L309D, Q311H, N434S (DHS) with or without terminal lysine and with or without an S228P mutation. In embodiments, Ab comprises an IgG₄ heavy chain comprising the mutations F234A, L235A (FALA) and T250Q, M428L (QL) with or without terminal lysine and with or without an S228P mutation. In embodiments, Ab comprises an IgG₄ heavy chain comprising the mutations L234S, L235T, G236R (STR) and T250Q, M428L (QL) with or without terminal lysine and with or without an S228P mutation.

The anti-GPC3 antibody disclosed herein has been engineered to facilitate efficient conjugation of the TOP1i linker-drug. The cysteine at position 214 of the light chain has been mutated to alanine, thereby disrupting the disulfide bridge that naturally forms with the cysteine at position 220 of the heavy chain (Eu numbering according to Kabat). This renders the cysteine at position 220 of the heavy chain free, or unpaired, and thus suitable for site-specific and controlled conjugation of the TOP1i linker-drug.

### 6.2.2. Number of Linked Drugs

In certain embodiments, the ADCs disclosed herein comprise drug molecules linked to antibody moieties in various stoichiometric molar ratios depending on the configuration of the antibody and, at least in part, the method used to effect conjugation.

The terms "drug load" or "drug loading" refer to the number of drug molecules per antibody in an individual ADC molecule. The number of TOP1i drugs linked to an anti-GPC3 ADC may vary and will be limited by the number of available attachments sites on the anti-GPC3 antibody. As contemplated for the anti-GPC3 ADCs of the invention, the linker will link a single TOP1i drug to the antibody in an anti-GPC3 ADC. As long as the anti-GPC3 ADC does not exhibit unacceptable levels of aggregation under the conditions of use and/or storage, anti-GPC3 ADCs (i.e., formula (IV)) having an n of up to 6 are contemplated. In certain embodiments, the anti-GPC3 ADCs have an n of in the range of from 1-6. In certain embodiments, the anti-GPC3 ADCs have an n selected from 1, 2, 3, 4, 5, or 6. In certain embodiments, n is 1, 2, 3, or 4. In certain embodiments, n is 2 or 4. In certain embodiments, n is 2. In certain embodiments, the drug loading may comprise 1 drug molecule, 2 drug molecules, 3 drug molecules, 4 drug molecules, 5 drug molecules, or 6 drug molecules.

Provided herein is a conjugation method of producing an anti-GPC3 ADC composition with the predominant species of ADC having an n of 2, and with the composition having a drug-antibody ratio (DAR) of 2 or about 2. The DAR is the average number of drugs linked to each antibody in the composition. Other methods of conjugation are known in the art and can be utilized to produce ADCs having different numbers of conjugated drugs (with an n of, e.g., 1, 2, 3, 4, 5, or 6) and compositions of different DARs (e.g., a DAR of 1, 2, 3, 4, 5, or 6).

### 6.2.3. Exemplary ADCs

In certain embodiments, an anti-GPC3 antibody drug conjugate of formula (IV) is provided: 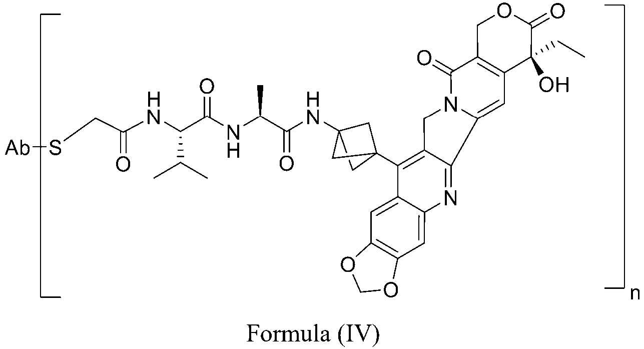 wherein antibody Ab comprises two heavy chains each having an amino acid sequence shown as SEQ ID NO:9 and two light chains each having an amino acid sequence shown as SEQ ID NO: 10. In certain embodiments, conjugation of the linker-drug to the antibody is via a linkage formed with a sulfhydryl group of a cysteine residue of the antibody. In certain embodiments, n has a value of 1, 2, 3, 4, 5, or 6.

In certain embodiments, n has a value of 1, 2, 4, or 6. In certain embodiments, n is 2. In certain embodiments, the anti-GPC3 ADC of structural formula (IV) comprises an Ab having a heavy chain having an amino acid sequence shown as SEQ ID NO: 9 and a light chain having an amino acid sequence shown as SEQ ID NO: 10 and n has a value of 1, 2, 4, or 6. In certain embodiments, the anti-GPC3 ADC of structural formula (IV) comprises an Ab having a heavy chain having an amino acid sequence shown as SEQ ID NO: 9 and a light chain having an amino acid sequence shown as SEQ ID NO: 10 and n has a value of 2.

In certain embodiments, an anti-GPC3 antibody drug conjugate of formula (IV) is provided: 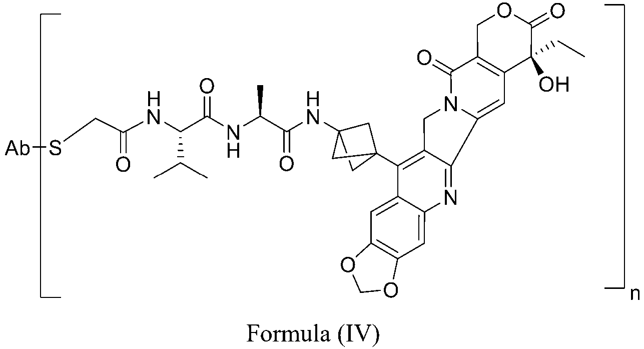 wherein antibody Ab comprises two heavy chains each having an amino acid sequence shown as SEQ ID NO: 11 and two light chains each having an amino acid sequence shown as SEQ ID NO: 10. In certain embodiments, conjugation of the linker-drug to the antibody is via a linkage formed with a sulfhydryl group of a cysteine residue of the antibody. In certain embodiments, n has a value of 1, 2, 3, 4, 5, or 6.

In certain embodiments, n has a value of 1, 2, 4, or 6. In certain embodiments, n is 2. In certain embodiments, the anti-GPC3 ADC of structural formula (IV) comprises an Ab having a heavy chain having an amino acid sequence shown as SEQ ID NO: 11 and a light chain having an amino acid sequence shown as SEQ ID NO: 10 and n has a value of 1, 2, 4, or 6. In certain embodiments, the anti-GPC3 ADC of structural formula (IV) comprises an Ab having a heavy chain having an amino acid sequence shown as SEQ ID NO: 11 and a light chain having an amino acid sequence shown as SEQ ID NO: 10 and n has a value of 2.

As used herein, "ADC-1" is a composition comprising ADCs according to structural formula (IV), wherein the Ab has two heavy chains each having an amino acid sequence shown as SEQ ID NO: 9 and two light chains each having an amino acid sequence shown as SEQ ID NO: 10 and a drug-antibody ratio (DAR) of about 2, with the predominant species having an n of 2. A procedure used to make ADC-1 is described in the Examples below. In an embodiment, ADC-1 is a composition comprising a plurality of ADCs according to structural formula (IV) with two or more different values of n. In some embodiments, the ADC-1 composition has an average drug-antibody ratio (DAR) of about 1.7, about 1.8, about 1.9, about 2.0, about 2.1, about 2.2, or about 2.3. In some embodiments, the ADC-1 composition of has an average of two molecules of the topoisomerase 1 inhibitor (Top1i) conjugated per anti-GPC3 antibody. In some embodiments, the predominant species of ADC in the ADC-1 composition has n = 2. In some embodiments, ADC-1 has an average DAR of about 2.

### 6.3. Methods of Use

In certain embodiments, methods for the treatment of hepatocellular carcinoma (HCC) comprising administering to a subject in need thereof a therapeutically effective amount of an ADC of formula (IV) are provided.

The term "subject," as used herein, refers to a human. The terms "human," "patient," and "subject" are used interchangeably herein.

The terms "treat," "treating," and "treatment," as used herein, refer to a method of alleviating or abrogating a disease and/or its attendant symptoms.

The phrase "therapeutically effective amount" refers to an amount of an ADC sufficient to prevent the development of or to alleviate to some extent one or more of the symptoms of the condition or disorder being treated when administered for treatment in a particular subject or subject population.

### 7. EXAMPLES

The following Examples, which highlight certain features and properties of the exemplary embodiments of the ADCs, antibodies, and TOP1i as described herein, are provided for purposes of illustration.

### Example 1: Snythesis of Linker-Drug

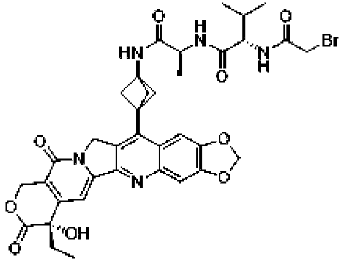

### Example 1

### (2S)-2-(2-bromoacetamido)-N-[(2S)-1-({3-[(7S)-7-ethyl-7-hydroxy-8,11-dioxo-7,8,11,13-tetrahydro-2H,10H-[1,3]dloxolo[4,5-g]pyrano[3',4':6,7]Indolizino[1,2-b]quinolin-14-yl]bicyclo[1.1.1]pentan-1-yl}amino)-1-oxopropan-2-yl]-3-methylbutanamide

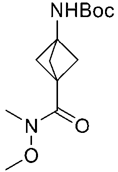

### Example 1A

### tert-butyl (3-(methoxy(methyl)carbamoyl)bicyclo[1.1.1]pentan-1-yl)carbamate

To a solution of 3-((*tert*-butoxycarbonyl)amino)bicyclo[1.1.1]pentane-1-carboxylic acid (4.9 g), *N*,*O*-dimethylhydroxylamine hydrochloride (2.2 g) and *N,N-*diisopropylethylamine (11.30 mL) in dichloromethane (10 mL) was added 1-[bis(dimethylamino)methylene]-1*H*-1,2,3-triazolo [4,5-*b*]pyridinium 3-oxide hexafluorophosphate (8.61 g) in portions at 10 °C. The reaction mixture was stirred at 20 °C for 12 hours. Two additional reactions were set up and allowed to stir at 20 °C for 12 hours as described. All three reactions were combined. The reaction was diluted with dichloromethane (200 mL) and added to 1 N aqueous HCl (50 mL). The precipitate formed was filtered, and the filtrate was allowed to separate. The organic layer was washed with saturated aqueous sodium bicarbonate solution (50 mL) and brine (50 mL), dried over anhydrous sodium sulfate, filtered, and the filtrate was concentrated under reduced pressure. The residue was purified by chromatography on silica gel, eluting with 1-50% ethyl acetate in petroleum ether to give the title compound. ¹H NMR (400 MHz, CDCl₃) *δ* ppm 4.97 (br s, 1H), 3.66 (s, 3H), 3.18 (s, 3H), 2.34 (s, 6H), 1.45 (s, 9H). MS (ESI+) *m*/*z* 271.2 (M+H)⁺.

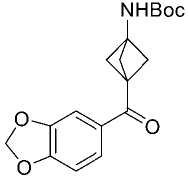

### Example 1B

### tert-butyl (3-(benzo[d][1,3]dioxole-5-carbonyl)bicyclo[1.1.1]pentan-1-yl)carbamate

To a solution of 5-bromobenzo[d][1,3]dioxole (8.83 g) in tetrahydrofuran (100 mL) was added n-butyllithium (17.57 mL, 2.5 M in hexane) slowly at -65 °C under nitrogen gas. The mixture was stirred at -65 °C for 30 minutes. A solution of Example 1A (4.75 g) in tetrahydrofuran (40 mL) was added slowly. The mixture was stirred at -65 °C for 3 hours. Three additional reactions were set up and allowed to stir at -65 °C for 3 hours. All four reactions were combined. The mixture was quenched with saturated aqueous ammonium chloride solution (500 mL) and extracted with ethyl acetate (3 × 500 mL). The combined organic layers were washed with brine (500 mL), dried over anhydrous sodium sulfate, filtered, and the filtrate was concentrated under reduced pressure. The residue was purified by chromatography on silica gel, eluting with 1-50% ethyl acetate in petroleum ether to give the title compound. ¹H NMR (400 MHz, CDCl₃) *δ* ppm 7.63 (dd, 1H), 7.45 (d, 1H), 6.82 (d, 1H), 6.03 (s, 2H), 5.04 (br s, 1H), 2.50 (s, 6H), 1.46 (s, 9H). MS (ESI+) *m*/*z* 354.2 (M+Na)⁺.

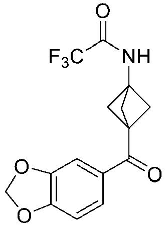

### Example 1C

### N-(3-(benzol[d][1,3]dioxole-5-carbonyl)bicyclo[1.1.1]pentan-1-yl)-2,2,2-trifluoroacetamide

Step 1: To a solution of Example 1B (6.2 g) in dichloromethane (62 mL) was added trifluoroacetic acid (62 mL) slowly at 0 °C. The reaction was stirred at 25 °C for 4 hours. Two additional reactions were set up and stirred at 25 °C for 4 hours. Each mixture was concentrated under reduced pressure. Each residue was used in the next step without further purification.

Step 2: To a solution of crude product above in dichloromethane (62 mL) was added N,N-diisopropylethylamine (16.34 mL) and trifluoroacetic anhydride (3.96 mL) dropwise at 0 °C. The mixture was stirred at 25 °C for 2 hours. Two additional reactions were set up as described and stirred at 25 °C for 2 hours. All three reactions were combined, poured into water (200 mL), and extracted with dichloromethane (2 × 200 mL). The organic layer was washed with brine (200 mL), dried over anhydrous sodium sulfate, filtered, and the filtrate was concentrated under reduced pressure. The residue was purified by column chromatography on silica gel, eluting with 25% ethyl acetate in petroleum ether to give the title compound. ¹H NMR (501 MHz, CDCl₃) *δ* ppm 7.61 (dd, 1H), 7.43 (d, 1H), 7.00 (s, 1H), 6.85 (d, 1H), 6.05 (s, 2H), 2.62 (s, 6H). MS (ESI+) *m*/*z* 328.2 (M+H)⁺.

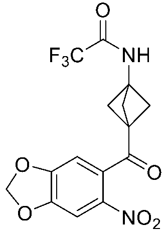

### Example 1D

### 2,2,2-trifluoro-N-(3-(6-nitrobenzo[d][1,3]dioxole-5-carbonyl)bicyclo[1.1.1]pentan-1-yl)acetamide

To a solution of Example 1C (4.3 g) in acetic anhydride (25 mL) was added copper(II) nitrate trihydrate (4.76 g) in portions at 0 °C. The mixture was stirred at 0 °C for 3 hours. Three additional reactions were set up and stirred at 0 °C for 3 hours as described. All four reactions were combined. The mixture was poured into water (50 mL) and extracted with ethyl acetate (5 × 100 mL). The organic layer was dried over anhydrous sodium sulfate, filtered, and the filtrate was concentrated under reduced pressure. The residue was purified by column chromatography on silica gel, eluting with 75% ethyl acetate in petroleum ether to give the title compound. ¹H NMR (400 MHz, CDCl₃) *δ* ppm 7.61 (s, 1H), 6.77 (br s, 1H), 6.64 (s, 1H), 6.21 (s, 2 H), 2.43 (s, 6 H). MS (APCI+) *m*/*z* 373.1 (M+H)⁺.

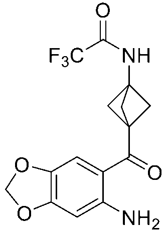

### Example 1E

### N-(3-(6-aminobenzo[d][1,3]dioxole-5-carbonyl)bicyclo[1.1.1]pentan-1-yl)-2,2,2-trifluoroacetamide

To a solution of Example 1D (4 g) in ethanol (40 mL) and water (8 mL), was added iron (5.4 g) and ammonium chloride (5.17 g) under nitrogen. The mixture was stirred at 100 °C for 3 hours. Three additional reactions were set up and stirred at 100 °C for 3 hours as described. After cooling to ambient temperature, all four reactions were combined. The mixture was poured into water (1 L) and extracted with ethyl acetate (5 × 500 mL). The combined organic phases were dried over anhydrous sodium sulfate, filtered, and the filtrate was concentrated under reduced pressure. The residue was purified by column chromatography on silica gel, eluting with 10-75% ethyl acetate in petroleum ether to give the title compound. ¹H NMR (400 MHz, CDCl₃) *δ* ppm 7.22 (s, 1H), 6.70 (s, 1H), 6.50 (s, 2H), 6.14 (s, 1H), 5.92 (s, 2H), 2.63 (s, 6H). MS (ESI+) *m*/*z* 343.2 (M+H)⁺.

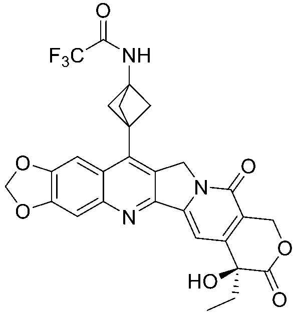

### Example 1F

### (S)-N-(3-(7-ethyl-7-hydroxy-8,11-dioxo-8,10,11,13-tetrahydro-7H-[1,3]dioxolo[4,5-g]pyrano[3',4":6,7]indolizino[1,2-b]quinolin-14-yl)bicyclo[1.1.1]pentan-1-yl)-2,2,2-trifluoroacetamide

To a suspension of Example 1E (3.5 g) and (*S*)-4-ethyl-4-hydroxy-7,8-dihydro-1*H-*pyrano[3,4-*f*]indolizine-3,6,10(4*H*)-trione (2.69 g) in toluene (140 mL) was added *para*toluenesulfonic acid monohydrate (1.945 g). The mixture was stirred at 115 °C for 12 hours. Three additional reactions were set up and stirred at 115 °C for 12 hours as described. After cooling to ambient temperature, all four reactions were combined. The mixture was filtered and the solid collected was triturated with acetonitrile (200 mL) to give the title compound. ¹H NMR (400 MHz, dimethyl sulfoxide *-d₆*) *δ* ppm 10.28 (s, 1H), 7.64 (s, 1H), 7.52 (s, 1H), 7.24 (s, 1H), 6.47 (s, 1H), 6.30 (dd, 2H), 5.42 (s, 2H), 5.36 (s, 2H), 2.87 (s, 6H), 1.94 - 1.77 (m, 2H), 0.88 (t, 3H). MS (ESI+) *m*/*z* 570.3 (M+H)⁺.

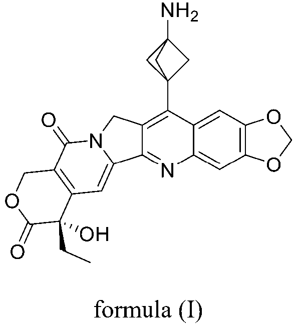

### Example 1G

### (7S)-14-(3-aminobicyclo[1.1.1]pentan-1-yl)-7-ethyl-7-hydroxy-2H,10H-[1,3]dioxolo[4,5-g]pyrano[3',4':6,7]indolizino[1,2-b]quinoline-8,11(7H,13H)-dione

To a solution of Example 1F (3 g) in methanol (30 mL) was added HCl (60 mL, 4 M in methanol). The mixture was stirred at 65 °C for 4 hours. Four additional reactions were set up and stirred at 65 °C for 4 hours as described above. After cooling to ambient temperature, all five reactions were combined. The mixture was concentrated under reduced pressure, and the residue was triturated with methanol (200 mL) to give the title compound. ¹H NMR (400 MHz, dimethyl sulfoxide *-d₆*) *δ* ppm 9.23 (s, 3H), 7.54 (s, 1H), 7.51 (s, 1H), 7.24 (s, 1H), 6.30 (d, 2H), 5.41 (s, 2H), 5.39 - 5.26 (m, 2H), 2.79 (s, 6H), 1.87 (hept, 2H), 0.88 (t, 3H). MS (ESI+) *m*/*z* 474.3 (M+H)⁺.

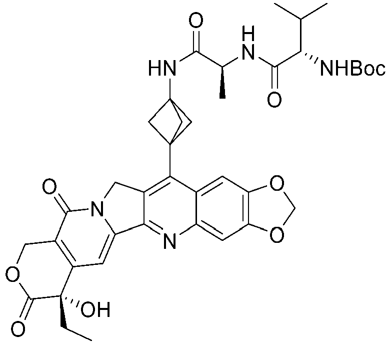

### Example 1H

### tert-butyl ((S)-1-(((S)-1-((3-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)bicyclo[1.1.1]pentan-1-yl)amino)-1-oxopropan-2-yl)amino)-3-methyl-1-oxobutan-2-yl)carbamate

To a suspension of (*S*)-2-((*S*)-2-((*tert*-butoxycarbonyl)amino)-3-methylbutanamido)propanoic acid (2.49 g), 2-hydroxypyridine 1-oxide (1.31 g), and *N*¹-((ethylimino)methylene)-*N*³,*N*³-dimethylpropane-1,3-diamine hydrochloride (2.26 g) in acetonitrile (40 mL), was added 2,6-lutidine (2.74 mL). The mixture was stirred at ambient temperature for 30 minutes. In a separate flask, Example 1G (4 g) and 2,6-lutidine (2.74 mL) were combined in *N*,*N*-dimethylformamide (40 mL) and the above solution was added. The mixture was stirred at ambient temperature overnight. The mixture was concentrated under reduced pressure and the residue was dissolved in dichloromethane (300 mL), washed with saturated aqueous ammonium chloride solution (100 mL) and brine (100 mL), dried over anhydrous sodium sulfate, and filtered. The filtrate was concentrated under reduced pressure. The residue was purified by column chromatography on silica gel, eluting with 0-10% methanol in dichloromethane to give the title compound. ¹H NMR (400 MHz, dimethyl sulfoxide *-d₆*) *δ* ppm 8.65 (s, 1H), 7.89 (d, 1H), 7.61 (s, 1H), 7.49 (s, 1H), 7.22 (s, 1H), 6.77 (d, 1H), 6.46 (s, 1H), 6.29 (d, 2H), 5.41 (s, 2H), 5.32 (s, 2H), 4.29 (q, 1H), 3.87 - 3.77 (m, 1H), 2.76 (s, 6H), 1.99 (q, 1H), 1.92 - 1.81 (m, 2H), 1.41 (s, 9H), 1.25 (d, 3H), 0.92 - 0.80 (m, 9H). MS (ESI+) *m*/*z* 744.4 (M+H)⁺.

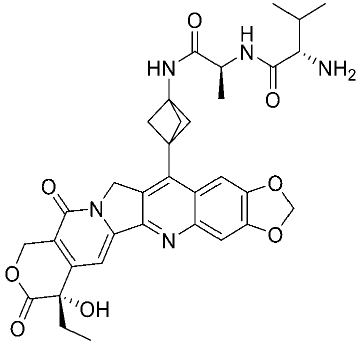

### Example 1I

### (S)-2-amino-N-((S)-1-((3-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)bicyclo[1.1.1]pentan-1-yl)amino)-1-oxopropan-2-yl)-3-methylbutanamide

Example 1H (5.5 g) was treated with trifluoracetic acid (30 mL) at ambient temperature for 30 minutes. The mixture was concentrated under reduced pressure and the residue was dissolved in 50% acetonitrile in water (200 mL). The solution was lyophilized to give the title compound. ¹H NMR (600 MHz, dimethyl sulfoxide-*d₆*) *δ* ppm 8.80 (s, 1H), 8.59 (d, 1H), 8.10 (d, 3H), 7.60 (s, 1H), 7.48 (s, 1H), 7.23 (s, 1H), 6.33 - 6.26 (m, 2H), 5.41 (d, 2H), 5.35 - 5.23 (m, 2H), 4.35 (p, 1H), 3.66 - 3.63 (m, 1H), 2.77 (s, 6H), 2.17 - 2.06 (m, 1H), 1.91 - 1.83 (m, 2H), 1.31 (d, 3H), 1.01 - 0.96 (dd, 6H), 0.89 (t, 3H). MS (ESI+) *m*/*z* 644.4 (M+H)⁺.

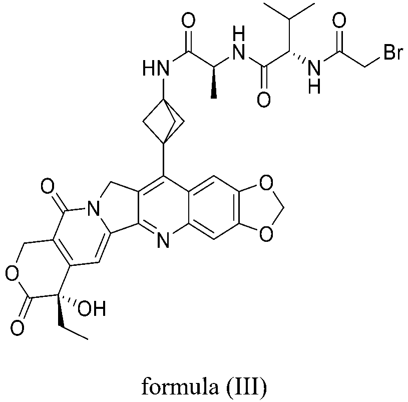

### Example 1J

### (2S)-2-(2-bromoacetamido)-N-[(2S)-1-(13-[(7S)-7-ethyl-7-hydroxy-8,11-dioxo-7,8,11,13-tetrahydro-2H,10H-[1,3]dioxolo[4,5-g]pyrano[3',4':6,7]indolizino[1,2-b]quinolin-14-yl]bicyclo[1.1.1]pentan-1-yl}amino)-1-oxopropan-2-yl]-3-methylbutanamide

To a solution of 2-bromoacetic acid (1.435 g) in *N,N*-dimethylformamide (26 mL) was added ethyl 2-ethoxyquinoline-1(2*H*)-carboxylate (2.55 g). The mixture was stirred at ambient temperature for 10 minutes. In a separate flask, Example 1I (4.5 g) and 2,6-lutidine (3.61 mL) were combined in *N*,*N*-dimethylformamide (26 mL), and the above solution was added. The mixture was stirred at ambient temperature for 30 minutes. The mixture was acidified with trifluoroacetic acid (4 mL) and purified by reverse-phase HPLC on a CombiFlash^{®} Teledyne Isco system using a Luna^{®} column (250 × 50 mm, 10 mm), eluting with 5-75% acetonitrile in water containing 0.1% trifluoroacetic acid over 30 minutes to give the title compound after lyophilization. ¹H NMR (600 MHz, dimethyl sulfoxide-*d₆*) *δ* ppm 8.57 (s, 1H), 8.32 (d, 1H), 8.16 (d, 1H), 7.67 (s, 1H), 7.52 (s, 1H), 7.24 (s, 1H), 6.30 (dd, 2H), 5.42 (s, 2H), 5.38 (d, 2H), 4.28 - 4.19 (m, 2H), 4.03 - 3.91 (m, 2H), 2.76 (s, 6H), 2.02 (h, 1H), 1.86 (ddp, 2H), 1.25 (d, 3H), 0.93 - 0.84 (m, 9H). MS (ESI+) *m*/*z* 764.46 (M+H)⁺.

### Example 2: Preparation and Purification of Antibody

mAb1, known as mAb hu GPC3 (LC:C214A) [hu IgG1/k] z, non-a, is a fully human IgG1 monoclonal antibody with a C214A mutation at the C-terminus of the light chain that enables site-specific and controlled linker-drug conjugation to Cysteine 220 on the Heavy Chain. The discovery and engineering of mAb1 is described below.

AMM-K C57BL/6 mice (AlivaMab^{®} Biologics, San Diego, CA, USA) which have human variable regions, were immunized with HEK-293 cells expressing cynomolgous macaque ("cyno") GPC3 (293H-cyno GPC3). After injection, plasma from immunized mice was screened by ELISA for binding to human GPC3.

From mice that tested plasma positive for human GPC3 antibodies, spleen cells were taken and fused with NS0 cells to generate the hybridomas, and hybridoma supernatants were screened for binding to endogenously expressed GPC3 on HepG2 cells, HEK-293H cells expressing human GPC3, or HEK-293H cells expressing cynomolgous monkey GPC3. A total of 57 hits from this screen were submitted for VH/VL sequence analysis. After sequence analysis and scrutiny of the matrixed assays that were performed, 27 antibodies were selected for recombinant production at a 35 mL scale and submitted for MMAE conjugation for a secondary cell death assay on HepG2 cells.

Recombinant proteins were screened in cell-based binding assays, cell death assays, and Biacore^{™} assays to determine which clones to scale up. Following final clone selection, the GPC3 antibody was re-cloned with a human IgG1, which was designated mAb1.

mAb1 was expressed in Chinese hamster ovary (CHO) cells as an IgG1 antibody with the heavy and light chain sequences of SEQ ID NO: 9 and SEQ ID NO: 10, respectively.

### Example 3: In vitro binding to GPC3

mAb1 binding affinity was characterized against both recombinant extracellular domain (ECD) and cell surface GPC3. Binding affinity was compared to GC33 (codrituzumab), which binds human GPC3 (Zhu, et al. 2013. First-in-man phase I study of GC33, a novel recombinant humanized antibody Against Glypican-3, in patients with advanced hepatocellular carcinoma. Clin. Cancer Res. DOI: 10.1158/1078-0432.CCR-12-2616).

mAb1 and GC33 binding to recombinant human GPC3 extracellular domain (ECD) was comparable by ELISA, EC₅₀ = 0.59 vs. 0.69 nM, respectively, with similar affinities observed for cyno GPC3. In contrast, binding affinity measured by FACS and SPR (Biacore) showed larger differences between mAb1 and GC33. By FACS, GC33 bound to cell surface GPC3 on four different cell types with higher affinity than mAb1. An even more dramatic difference was observed using SPR, with GC33 having a *K*_{D} = 2.9 nM vs. 121 nM for mAb1 (Table 1). Collectively, these data indicate GC33 has a higher affinity for GPC3 than mAb1.

**Table 1. Apparent Binding Affinity of mAb1 to Recombinant and Cell Surface Human GPC3.**

| Assay | Cell line/Species | GC33 | mAb1 |
|---|---|---|---|
| Biacore *K_{D}* (nM) | Human GPC3 ECD | 2.9 | 121 |
| | Cyno GPC3 ECD | 2.6 | 132 |
| ELISA EC50 (nM) | Human GPC3 ECD | 0.69 | 0.59 |
| | Cyno GPC3 ECD | 0.81 | 0.53 |
| FACS EC50 (nM) | HEK-293-human GPC3 | 2.3 | 10.05 |
| | HEK-293-cyno GPC3 | 1.27 | 6.35 |
| | Hep3B | 1.17 | 8.75 |
| | H1581 | 1.13 | 7.24 |

### Methods:

Cell Culture: All cells were cultured at 37°C with 5% CO2. HCC cell lines HepG2, Hep3B and SK-Hep1 were obtained from American Type Culture Collection (ATCC). HepG2 cells were maintained in MEM (Gibco, Cat. No. 11095-080) supplemented with 10% fetal bovine serum (FBS; Gibco, 10082-147). To make SK-Hep1 cells stably expressing-GPC3 cells, DNA encoding full length human GPC3 (NM_004484.4) was synthesized at GeneWiz and subcloned into pLVX-IRES-Puro lentivirus vector (Clontech, Cat. No. 632183). Lentivirus was generated by transfecting LentiX-293T cells (Clontech, Cat. No. 632180) with either pLVX-IRES-Puro empty vector or pLVX-IRES-Puro containing human GPC3 using Lenti-X packaging single shots (Clontech, Cat. No. 631276). Supernatant containing virus was collected and transduced into SK-Hep1 cells using Lenti-X accelerator (Clontech, Cat. No. 631256). After transduction, cells were treated with 1µg/mL puromycin (Sigma, 540411) and FACS sorted to generate stable empty vector or GPC3-positive cells. SK-Hep1 empty vector and SK-Hep1-GPC3 cells were maintained in MEM (Gibco, 10082-147) supplemented with 10% FBS and 1µg/mL puromycin (Sigma, 540411 - puromycin was removed during proliferation assays).

HEK-293 cells were obtained from ATCC, maintained in RPMI base media supplemented with 10% FBS. To make HEK-293 cells overexpressing human GPC3 or GPC3 from potential toxicology species, DNA encoding full length human (NM_004484.4), cynomolgus monkey (XM_005594608.4), murine (NM_016697.3) or rat (NM_012774.2) GPC3 was synthesized and subcloned into pLVX-IRES-Puro lentivirus vector at GeneWiz. Generation of lentivirus and transduction of HEK-293 cells followed an identical protocol to SK-Hep1 cells described above, with selection of GPC3 positive cells following continuous treatment with 1 µg/mL puromycin (Sigma, 540411). All the cells were cultured at 37 °C with 5% CO₂.

HCC cell lines were dissociated from storage flasks using 5 mL Cell Dissociation Solution, Non-enzymatic, in PBS (Sigma, C5914-100ML). Cells were filtered through pre-separation filters of 20 µM (Miltenyi Biotec, Cat. No. 130-101-812) before counting of live cells. 1000 cells were plated into each well of a black 96-well plate (Corning, 3904) or 300 cells/well of a white 384-well plate (Corning, 3765) and briefly spun at 800 rpm for 10 seconds. For 3D proliferation assays, 5000 cells were plated into each well of a Black/Clear Round Bottom Ultra-Low Attachment Surface Spheroid 96 well Microplate (Corning, 4520) and briefly spun at 800 rpm for 10 seconds. Using a Tecan D300e Digital Dispenser, a 3-fold dose response using a stock concentration of ADC containing surfactant (0.3% Tween 20) or DMSO solution for payload, was applied to the cells and incubated for an additional 10 days (ADC and payload) at 37°C in 5% CO2. The dose response started at 100 nM of ADC. Samples were run in duplicates. Cell viability was assessed with the CellTiter-Glo^{®} Luminescent Cell Viability Assay (Promega, Cat. No. G7573), according to the manufacturer's instructions, with the exception that the plates were shaken for 30 minutes prior to reading. Luminescence was read on the BioTek Synergy Neo2, and data was analyzed using GraphPad Prism software. Percent inhibition was calculated compared to a positive control (staurosporine) and negative control (vehicle treated). The calculation used was as follows: 100*(Vehicle - ADC)/(Vehicle - staurosporine). Half-maximal inhibition values (IC50) were calculated using GraphPad Prism Software, where n = 3 independent biologic replicates.

HEK-293 parental and stable GPC3 cells were washed with 5 mL Cell Dissociation Solution, Non-enzymatic, in PBS (Sigma, C5914-100ML) for 5 minutes at 37°C. Media was added to the dissociated cells and they were counted. 300 cells were plated out into each well of a white 384-well plate and briefly spun at 800 x g for 10 seconds. All plates were treated similarly to above, for a total of 3 days.

Binding Enzyme-Linked Immunosorbant Assay (ELISA): For mAb1 and ADC-1 binding ELISA, 96-well Pierce^{™} nickel coated plates (ThermoFisher) were coated with 0.1 µg/ml His tagged human, cyno, mouse, or rat GPC3 extracellular domain (ECD), 50 µl/well for 1 hour under ambient conditions. Following incubation with GPC3 ECDs, plates were washed 3 times with phosphate-buffered saline containing 0.05% Tween 20 (PBS-T) and blocked 1 hour at room temp with 300 µl 2% goat serum/phosphate-buffered saline (Jackson ImmunoResearch, West Grove, PA, USA). After blocking for an hour, plates were again washed 3X with PBS-T and then with a 1:3 dose titrations of ADC/Ab in blocking buffer, starting at 66.67 nM, were added to duplicate wells for 1 hour under ambient conditions. Plates were again washed 3X in PBS-T and an incubation of blocking buffer containing 1:10,000 HRP goat-anti human IgG (Jackson Immuno) for 30 minutes at room temp. After washing plates 3X with PBS-T, 50 ul/well of Ultra TMB substrate (ThermoFisher) was added and incubated 5-10 minutes protected from light, after which 50 ul/well of 1 N HCl STOP solution was added and absorbance OD₄₅₀ reading were measured on a Synergy Neo2 plate reader (Biotek Instruments, Winooski, VT, USA) using Gen5 3.10 software.

Surface Plasmon Resonance Binding Assay (SPR): A Biacore^{™} T200 SPR instrument (Cytiva, Marlborough, MA, USA) was used to measure the binding kinetics of human, cynomolgus, mouse and rat GPC3 as an analyte (Cytiva) binding to mAb1 and its ADC as ligands. The assay format was based on fragment crystallizable antibody effector region (Fc) capture via immobilized with anti-human Fc (Pierce ThermoFisher). A standard amine coupling protocol was employed to immobilize the capture reagents via primary amines to the carboxy-methyl dextran surface of CM5 sensor chips (Cytiva); capture antibodies were coupled to a level of approximately 2000 relative units (RU). For binding kinetic measurements, the assay buffer was HBS-EP+ (10 mM HEPES, pH 7.4, 150 mM NaCl, 3 mM EDTA, 0.05% Polysorbate 20). During the assay, all measurements were referenced against the capture surface alone. Each assay cycle consisted of the following steps: (1) Capture of ligand to approximately 80 RU; (2) Analyte injection over both reference and test surface, 240 µL at 80 µL/minute, after which the dissociation was monitored for 15 minutes at 80 µL/minute; (3) Regeneration of capture surface with 10 mM glycine, pH 1.5. For kinetic determinations, analyte injections were a 3-fold dilution series starting at 900 nM for a total of five concentrations; buffer only injections were included for secondary referencing. Data were processed and fit to a 1:1 binding model using Biacore^{™} T200 Evaluation Software to determine the binding kinetic rate constants, *kₐ* (on-rate) and *k_{d}* (off-rate), and the equilibrium dissociation constant (affinity, *K*_{D}).

Fluorescence Activated Cell Sorting (FACS) Analysis: Cells used for FACS included HEK-293 cell stably transduced with either human, cynomolgus, rat or mouse GPC3. Cells were harvested using 5 mL Cell Dissociation Buffer, Enzyme-Free (MilliporeSigma) and counted using a ViCell XR cell viability analyzer (BeckmanCoulter, Indianapolis, IN, USA). A minimum viability of 2x10⁶ cells/mL were used. Cells were then centrifuged at 800 *x* g for 5 minutes and resuspended in cold FACS buffer (5% FBS/PBS with 0.1% sodium azide). 50 µL of a 2X concentration of mAb/ADC (final concentrations are indicated in the figures) was added to U-bottom 96-well plates. 50 µL of cells (100,000 cells) were added and plates were incubated at 4 °C for one hour. Next, each well was washed three times with 150 µL of cold FACS buffer by centrifuging at 320 *x* g for five minutes. After the third wash, cells were resuspended in 50 µL of FACS buffer containing 8 µg/mL of Alexa Fluor 488 goat anti-human IgG (H+L) secondary (Invitrogen ThermoFisher) and 1:1000 Live/Dead exclusion dye (ThermoFisher). Cells were mixed and incubated at 4 °C for 1 hour in the dark. The cells were then washed 3X with FACS buffer and fixed with 100 µL of 1% formaldehyde in cold PBS (Polysciences, Niles, IL, USA). Cells were analyzed on a Becton Dickinson FACSCanto II flow cytometer (Becton Dickinson, Franklin Lakes, NJ, USA). Data was analyzed using FlowJo^{™} v10.10 flow cytometry analysis software (Becton Dickinson.

### Example 4. Preparation of ADC-1

mAb1 was conjugated to linker-drug (Example 1J) as described below to form antibody drug conjugate ADC-1.

The mAb1 antibody from Example 2 was partially reduced with an excess of reducing agent and then conjugated with an excess of drug-linker. This results in the final drug product comprising an ADC primarily containing two drug molecules, i.e., an ADC of formula (IV) with an n of 2 and a DAR of 2 or about 2.

A solution of mAb1 antibody at 4 °C was treated with PBSE (potassium phosphate dibasic/sodium chloride/ethylenediaminetetraacetic acid buffer; 125 mM potassium phosphate, 150 mM NaCl, 6.3 mM EDTA, pH 7.2) to achieve a final concentration of 0.1 mM Tris (2-carboxyethyl) phosphine (TCEP, 10 mM, 10 molar equivalents, Bond Breaker^{™}, ThermoFisher) was added to the mAb1 antibody solution (approximately 10~20 mg/mL in 0.5X PBSE with gentle stir plate mixing (225 rpm) for 5 minutes. Following incubation at 4 °C for 20 hours, the solution was brought to 22 °C.

UFDF (ultrafiltration/difiltration) was applied to buffer exchange the reduced mAb antibody into PBSE buffer using a Pellicon^{®} 3 0.57 m² Cassette (MilliporeSigma, Burlington, MA, USA). Ultrafiltration (UF) was performed until the mAb antibody reached a concentration of 40 mg/mL. Diafiltration (DF) was performed with 10 diavolumes (DV) of PBSE.

A 6.6 molar equivalent of 10 mM solution of linker-drug (Example 1J) in *N,N-*dimethylacetamide with gentle stir plate mixing was incubated at 23 °C for 16 hours. Following incubation, 8.0 equivalents of 100 mM *N*-Acetyl-L-cysteine (NAC, MilliporeSigma) prepared in sterile purified water, were added to the solution, gently mixed, followed by 2-hour incubation at 23 °C.

UFDF was applied to buffer exchange the resulting antibody drug conjugate (ADC-1) into a mixture of dimethyl sulfoxide (dimethyl sulfoxide) and 15 mM histidine buffer, pH 6, and thereafter into a 15 mM histidine buffer using a Pellicon^{®} 3 0.57 m² Cassette (MilliporeSigma). Ultrafiltration and diafiltration were performed. The ADC-1 solution was collected then further diluted with buffer. The final ADC-1 solution was sterile filtered with a 0.22 µm filter and stored at -80 °C.

### Example 5. Comparative efficacy of TOP1i drug and linker-drug construct

The anti-proliferative activity of mAb1 and GC33 as antibody-drug conjugates was tested after conjugation to the linker-drug in Example 1J, using MSL-109 ADCs as a non-targeting control (anti-CMV antibody; Dobryski et al, 1991). SK-Hep1 cells expressing human GPC3, HepG2 cells, and Hep3B cells in 2D cell culture were treated with a 1:3 dilution of a DAR = 2 or DAR = 6 version of ADC.

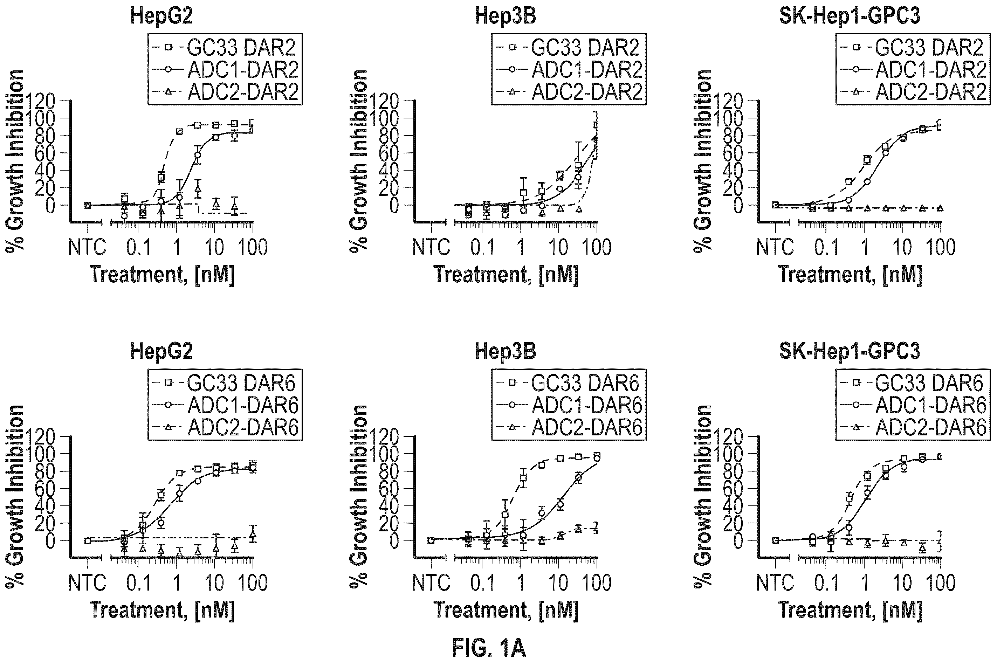

As shown in Tables 2-4 and FIG. 1A, GC33 ADCs (DAR2 and DAR6) had greater anti-proliferative activity against all three GPC3 expressing cell lines when grown as a monolayer (2D) than mAb1 ADCs. As expected, little to no activity was observed with the MSL109 ADCs.

**Table 2. 2D growth inhibition IC50 values for GPC3-expressing SK-Hep1 cells.**

| Antibody | GC33 ADC | mAb1 ADC | MSL ADC |
|---|---|---|---|
| DAR2 | 1.24 | 2.78 | >100.0 |
| DAR6 | 0.48 | 0.89 | >100.0 |

**Table 3. 2D growth inhibition IC50 values for HepG2 cells.**

| Antibody | GC33 ADC | mAb1 ADC | MSL ADC |
|---|---|---|---|
| DAR2 | 0.54 | 3.05 | >100.0 |
| DAR6 | 0.35 | 1.03 | >100.0 |

**Table 4. 2D growth inhibition IC50 values for Hep3B cells.**

| Antibody | GC33 ADC | mAb1 ADC | MSL ADC |
|---|---|---|---|
| DAR2 | 23.99 | 53.06 | 74.79 |
| DAR6 | 0.70 | 12.84 | >100.0 |

The DAR = 2 versions of the GC33 ADC and the DAR = 2 version of the mAb1 ADC (ADC-1) were also tested in spheroid 3D cell culture systems. Spheroid 3D cultures are generated by culturing cancer cell lines in non-adherent plates, whereby the cells aggregate to form a spheroid. Cancer cell line spheroids are hypothesized to better mimic the structural and physiological characteristics of tumors with respect to drug delivery and therapeutic response compared to 2D conditions. (Kapalczynska et al, 2D and 3D cell cultures - a comparison of different types of cancer cell cultures. Arch Med Sci. 2018 Jun;14(4):910-919, doi: 10.5114/aoms.2016.63743).

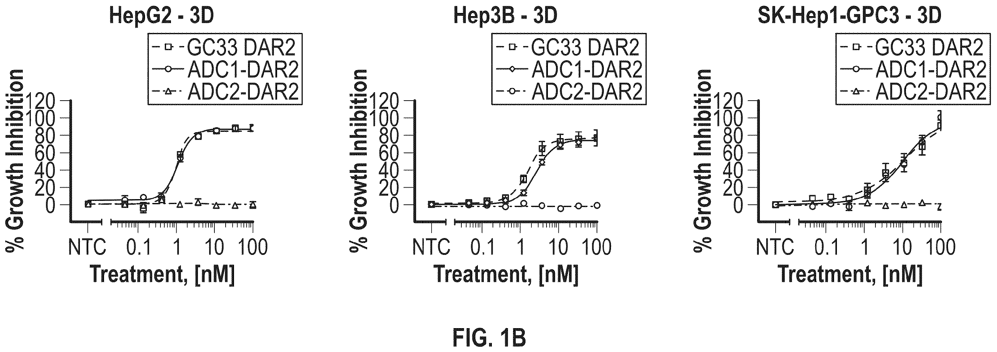

The anti-proliferative activity of DAR2 GC33 ADC, ADC-1 (DAR2), and DAR2 MSL ADC was tested on SK-Hep1 cells expressing human GPC3, HepG2 cells, and Hep3B cells grown as spheroids in 3D cell culture. Surprisingly, ADC-1 and GC33 ADC demonstrated similar anti-proliferative activity against cells grown in 3D (Table 5, FIG. 1B). This result was unexpected in view of GC33 ADC's superior inhibition in 2D cell culture systems (Tables 2-4, FIG. 1A), and binding to cells (Table 1).

**Table 5. IC50 values in 3D spheroid cells.**

| ADC | HepG2 | Hep3B | SK-Hep1-GPC3 |
|---|---|---|---|
| DAR2 GC33 | 1.07 | 2.21 | 10.36 |
| ADC-1 | 1.19 | 3.92 | 10.61 |
| DAR 2 MSL ADC | >100.0 | >100.0 | >100.0 |

Finally, ADC-1 (DAR2) and DAR2 GC33 ADC anti-tumor activity were compared in a human HCC Hep3B cell-line derived xenograft (CDX) mouse model. Three million cells per mouse were inoculated subcutaneously into the right flank of female CB17 SCID mice. Mouse tumors were size matched 21 days after inoculation. The mean tumor volume at size matching was approximately 215 mm³ with a range of 159 to 271 mm³.

ADC-1 or DAR2 GC33 were administered intraperitoneally at a low or medium dose as a single bolus and compared to the non-targeting MSL ADC control ("ADC-2"). As shown in FIG. 1C (low dose) and FIG. 1D (medium dose), similar tumor growth inhibition and tumor growth delay were observed in mice treated with either ADC-1 or DAR2 GC33.

### Example 6. Testing of GC33 ADC, ADC-1 and ADC-1a in non-human primates

To determine toxicity and toxicokinetics of the ADCs, studies were undertaken with the non-human primate (NHP) cynomolgus monkeys dosed at Q3W for 4 weeks (2 doses).

Based on other ADCs using the linker-drug shown in Example 1J, an ADC with a DAR of 6 was expected to show superior efficacy and safety at a moderate dose. However, treatment at a medium dose of the DAR6 GC33 ADC showed strongly adverse events, including gastrointestinal epithelial degradation leading to mortality of the animals tested by day 9 (Table 6). Similar toxicities were observed with a low dose of DAR2 GC33 ADC leading to mortality of the animals by day 9.

In contrast, ADC-1 (DAR2 mAb1 ADC) was well tolerated, at both low and medium, with no significant clinical signs observed.

In view of the superior tolerability and comparable efficacy, ADC-1 was selected for further in vivo studies.

**Table 6: ADC Toxicity**

| Group | Test Item | DAR | Dose* | Frequency | In life study observations (day 27) |
|---|---|---|---|---|---|
| 1 | Control | - | 0 | Q3W | None |
| 2 | ADC-1 | 2 | 2x | Q3W | Well tolerated |
| 3 | ADC-1 | 2 | 3x | Q3W | Some loss of BW (4-8%), but no significant clinical signs |
| 5 | DAR2 | 2 | 2x | Q3W | 2/2 euthanized on day 9 due to adverse events |
| | GC33 | | | | |
| 6 | DAR6 | 6 | 3x | Q3W | 3/3 euthanized on day 9 due to adverse events |
| | GC33 | | | | |

| | | | | | |
|---|---|---|---|---|---|
| *dose description are matched by total drug payload | | | | | |

### Example 7. Generation of Tumor Bearing Mice and Determination of Tumor Volume of Subcutaneous Flank Tumors

To generate cell line derived xenografts, tumor cells were inoculated subcutaneously into the right flank of female CB17 SCID mice (Charles River Laboratories, Wilmington, MA, USA) with NCI-H1581 cells (ATCC) or Hep3B cells, or female SCID-Beige mice (Charles River) with HepG2 cells. The injection volume was 0.1 mL composed of cells suspended in a 1:1 mixture of S-MEM (ThermoFisher) and Matrigel (Becton Dickenson, Franklin Lakes, NJ, USA). Animals with tumors were randomized at volumes ranging from 158 to 335 mm³ prior to treatment.

To generate LI1068 and LI6610 in vivo PDX models, tumors from the previous lineage were excised from mice and tumor fragments of 2-3 mm in diameter were subcutaneously implanted into the right flank of female Balb/c nude mice (GemPharmatech, Nanjing, CN). The animals carrying tumors were randomized at volumes ranging from 101 to 151mm³.

Therapy began within 24 hours following randomization of animals carrying tumors into required cohorts. Tumor volume was estimated once or twice weekly. Measurements of length (L) and width (W) of the tumor were obtained via electronic calipers and volume was calculated according to the following equation: V = (L X W²)/2.

In the following studies, ADC-2 refers to a DAR2 ADC using MSL109 conjugated to linker-drug (Example 1J) and is used as a non-targeting control antibody/negative control ADC.

### Study 1. Growth Inhibition of Xenografted Human NSCLC (NCI-H1581) by Various Doses of ADC-1

NCI-H1581 cells were grown to passage 3 in vitro. Five million cells per mouse were inoculated subcutaneously into the right flank of female CB17 SCID mice. Mouse tumors were size matched 13 days after inoculation. The mean tumor volume at size matching was approximately 211 mm³ with a range of 158 to 335 mm³.

Efficacy of ADC-1 was determined in subcutaneous xenografts of NCI-H1581 cells derived from a human NSCLC tumor sample. ADC-1 or ADC-2 was administered intraperitoneally at low (1x), medium (2x) and high (3x) doses as a single bolus in 8 mice at each dose level and, compared to vehicle controls, induced statistically significant (p < 0.05) TGI (85, 92 and 95% respectively on Day 11 post dosing) and TGD (100, 218 and 318% respectively) at all dose levels tested. A medium dose level of ADC-1 elicited an OR of 63% (63% PR) while a high dose level of ADC-1 elicited an OR of 100% (50% CR, 50% PR) (Table 7). The mice tolerated all treatments.

**Table 7. Sensitivity of NSCLC flank tumors to ADC treatment.**

| **Treatment** | **Dose** | **Dosing Regimen** | **TGI (%)** | **TGD (%)** | **CR (%)** | **PR (%)** | **OR (%)** |
|---|---|---|---|---|---|---|---|
| Vehicle | 0 | QD x 1 | - | - | 0 | 0 | 0 |
| ADC-2 | Low | QD x 1 | 17 | 0 | 0 | 0 | 0 |
| ADC-2 | Medium | QD x 1 | 67** | 82** | 0 | 0 | 0 |
| ADC-2 | High | QD x 1 | 91** | 136** | 0 | 0 | 0 |
| ADC-1 | Low | QD x 1 | 85** | 100** | 0 | 0 | 0 |
| ADC-1 | Medium | QD x 1 | 92** | 218** | 0 | 63 | 63 |
| ADC-1 | High | QD x 1 | 96** | 318** | 50 | 50 | 100 |

| | | | | | | | |
|---|---|---|---|---|---|---|---|
| TGI (%) is percent tumor growth inhibition; TGD (%) is percent tumor growth delay; CR (%) is percent complete response rate; PR (%) is percent partial response rate; OR (%) is the percent overall response rate; * p<0.05 when compared to the vehicle control group; **= p < 0.01 when compared to the vehicle control group | | | | | | | |

### Study 2. Growth Inhibition of Xenografted Human HCC (Hep3B) by Various Doses of ADC-1

Hep3B cells were grown to passage 3 in vitro. Three million cells per mouse were inoculated subcutaneously into the right flank of female CB 17 SCID mice. Mouse tumors were size matched 21 days after inoculation. The mean tumor volume at size matching was approximately 215 mm³ with a range of 159 to 271 mm³.

Efficacy of ADC-1 was determined in subcutaneous xenografts of Hep3B cells, derived from human Hepatocellular cancer. ADC-1 was administered intraperitoneally at low (1x), medium (2x) and high (3x) doses as a single bolus and compared to vehicle controls, induced statistically significant (p < 0.05) TGI (54, 84 and 98% respectively) on Day 24 post dosing, and TGD (52, 295 and >400% respectively) at all dose levels tested. A medium dose level of ADC-1 elicited an OR of 75% (63% CR, 13% PR) while a high dose level elicited an OR of 100% (100% CR). (Table 8). The mice tolerated all treatments.

**Table 8. Sensitivity of HEP3B flank tumors to ADC-1 treatment.**

| **Treatment** | **Dose** | **Dosing Regimen** | **TGI (%)** | **TGD (%)** | **CR (%)** | **PR (%)** | **OR (%)** |
|---|---|---|---|---|---|---|---|
| Vehicle | 0 | QD x 1 | - | - | 0 | 0 | 0 |
| ADC-2 | High | QD x 1 | 4 | 14 | 0 | 0 | 0 |
| ADC-1 | Low | QD x 1 | 54** | 52* | 0 | 0 | 0 |
| ADC-1 | Medium | QD x 1 | 84** | 295** | 63 | 13 | 75 |
| ADC-1 | High | QD x 1 | 98** | >400** | 100 | 0 | 100 |

| | | | | | | | |
|---|---|---|---|---|---|---|---|
| TGI (%) is percent tumor growth inhibition; TGD (%) is percent tumor growth delay; CR (%) is percent complete response rate; PR (%) is percent partial response rate; OR (%) is the percent overall response rate; * p<0.05 when compared to the vehicle control group; **= p < 0.01 when compared to the vehicle control group | | | | | | | |

### Study 3: Growth Inhibition of Xenografted Human HCC (HepG2) by Various Doses of ADC-1

HepG2 cells were grown to passage 3 in vitro. Five million cells per mouse were inoculated subcutaneously into the right flank of female SCID-Beige mice. Mouse tumors were size matched 15 days after inoculation. The mean tumor volume at size matching was approximately 226 mm³ with a range of 191 to 256 mm³.

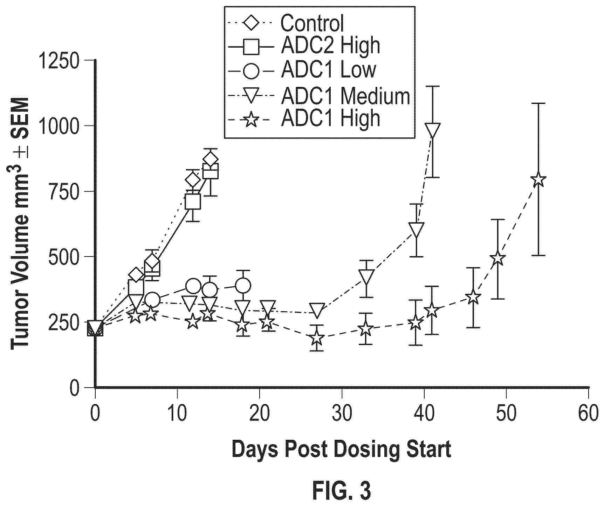

Efficacy of ADC-1 was determined in subcutaneous xenografts of HepG2 cells, derived from human HCC. ADC-1 was administered intraperitoneally at low (1x), medium (2x), and high (3x) doses as a single bolus and compared to vehicle controls, induced statistically significant (p < 0.05) TGI (58, 64 and 84% respectively) on Day 14 post dosing, and TGD (>50, 128 and 244% respectively) at all tested dose levels. At the high dose level, ADC-1 treatment elicited an OR of 20% (20% PR) (Table 9 and Figure 3). The mice tolerated all treatments.

**Table 9. Sensitivity of HEPG2 flank tumors to ADC-1 treatment.**

| Treatment | **Dose** | **Dosing Regimen** | **TGI (%)** | **TGD (%)** | **CR (%)** | **PR (%)** | **OR (%)** |
|---|---|---|---|---|---|---|---|
| Vehicle | 0 | QD x 1 | - | - | 0 | 0 | 0 |
| ADC-2 | High | QD x 1 | 6* | 0 | 0 | 0 | 0 |
| ADC-1 | Low | QD x 1 | 58** | >50** | 0 | 0 | 0 |
| ADC-1 | Medium | QD x 1 | 64** | 128** | 0 | 0 | 0 |
| ADC-1 | High | QD x 1 | 84** | 244** | 0 | 20 | 20 |

| | | | | | | | |
|---|---|---|---|---|---|---|---|
| TGI (%) is percent tumor growth inhibition; TGD (%) is percent tumor growth delay; CR (%) is percent complete response rate; PR (%) is percent partial response rate; OR (%) is the percent overall response rate; * p<0.05 when compared to the vehicle control group; **= p < 0.01 when compared to the vehicle control group | | | | | | | |

### Study 4. Growth Inhibition of Patient Derived Xenografts (LI1068) by Various Doses of ADC-1

Following growth in the right flank of stock mice, tumor fragments of LI1068 approximately 2-3 mm in diameter were subcutaneously implanted in the right flank of female Balb/c nude mice. Tumors were size matched 37 days following inoculation. The mean tumor volume at size matching was approximately 132 mm³ with a range of 101 to 151 mm³.

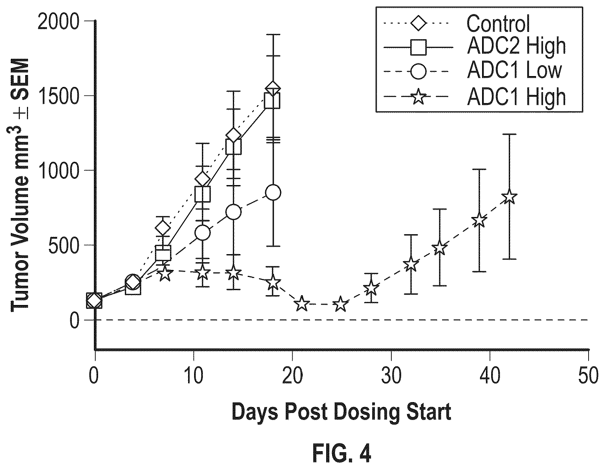

Efficacy of ADC-1 was determined in a subcutaneous PDX model of hepatocellular carcinoma. ADC-1 was administered intraperitoneally to five mice per group at low (1x) or high (3x) doses as a single bolus. Compared to vehicle controls, the high dose of ADC-1 induced a statistically significant (p < 0.05) TGI (83% on Day 18 post dosing) and TGD (382%) with a 40% CR rate observed (Table 10 and Figure 4). The mice tolerated all treatments.

**Table 10. Growth inhibition in HCC patient derived xenograft models following treatment with ADC-1.**

| Treatment | **Dose** | **Dosing Regimen** | **TGI (%)** | **TGD (%)** | **CR (%)** | **PR (%)** | **OR (%)** |
|---|---|---|---|---|---|---|---|
| Vehicle | 0 | QDx1 | - | - | 0 | 0 | 0 |
| ADC-2 | High | QD x 1 | 5 | 27 | 0 | 0 | 0 |
| ADC-1 | Low | QD x 1 | 45 | 155 | 0 | 0 | 0 |
| ADC-1 | High | QD x 1 | 83* | 382** | 40 | 0 | 40 |

| | | | | | | | |
|---|---|---|---|---|---|---|---|
| TGI (%) is percent tumor growth inhibition; TGD (%) is percent tumor growth delay; CR (%) is percent complete response rate; PR (%) is percent partial response rate; OR (%) is the percent overall response rate; * p<0.05 when compared to the vehicle control group; **= p < 0.01 when compared to the vehicle control group | | | | | | | |

### Study 5: Growth Inhibition of Patient Derived Xenografts (LI6610) by Various Doses of ADC-1

Following growth in the right flank of stock mice, tumor fragments of LI6610 approximately 2-3 mm in diameter were subcutaneously implanted in the right flank of female Balb/c nude mice. Tumors were size matched 20 days following inoculation. The mean tumor volume at size matching was approximately 119 mm³ with a range of 103 to 135 mm³.

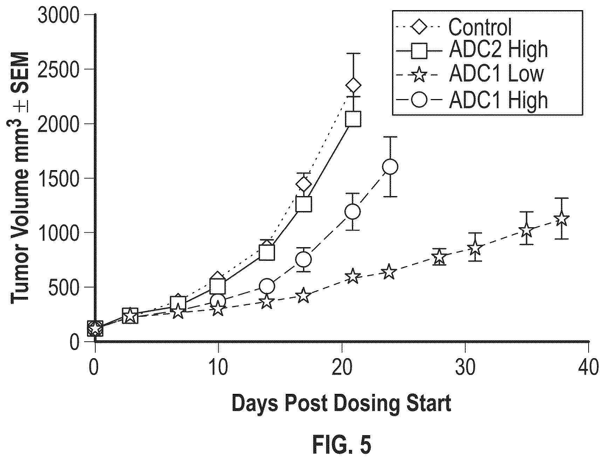

Efficacy of ADC-1 was determined in a subcutaneous PDX model of HCC L16610. ADC-1 was administered intraperitoneally at low (1x) or high (3x) doses as a single bolus, and, compared to vehicle controls, induced a statistically significant (p < 0.05) TGI (49 and 74% respectively) on Day 21 post dosing and TGD (24 and 147% respectively) (Table 11 and Figure 5). The mice tolerated all treatments.

**Table 11. Growth inhibition in HCC patient derived xenograft models following treatment with ADC-1.**

| **Treatment** | **Dose** | **Dosing Regimen** | **TGI (%)** | **TGD (%)** | **CR (%)** | **PR (%)** | **OR (%)** |
|---|---|---|---|---|---|---|---|
| Vehicle | 0 | Q21D x 2 | - | - | 0 | 0 | 0 |
| ADC-2 | High | Q21D x 2 | 13 | 0 | 0 | 0 | 0 |
| ADC-1 | Low | Q21D x 2 | 49** | 24* | 0 | 0 | 0 |
| ADC-1 | High | Q21D x 2 | 74** | 147** | 0 | 0 | 0 |

| | | | | | | | |
|---|---|---|---|---|---|---|---|
| TGI (%) is percent tumor growth inhibition; TGD (%) is percent tumor growth delay; CR (%) is percent complete response rate; PR (%) is percent partial response rate; OR (%) is the percent overall response rate; * p<0.05 when compared to the vehicle control group; **= p < 0.01 when compared to the vehicle control group | | | | | | | |

| **Table 12. SEQUENCE LISTING TABLE** | | |
|---|---|---|
| SEQ ID NO: | Description | Sequence |
| 1 | Ab heavy chain variable sequence | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSS |
| 2 | Ab light chain variable sequence | DIQLTQSPSFLSASVGDRVTITCRASQDISSYL AWYQQEPGKAPKLLIYATSTLQSGVPSRFSG SGSGTEFTLTITSLQPEDFATYYCQQLNSYPW TFGQGTKVEIK |
| 3 | CDR-H1 | GFTFSGFGMN |
| 4 | CDR-H2 | YTSSSSRTIYYADSVKG |
| 5 | CDR-H3 | DSSGWYDAFDI |
| 6 | CDR-L1 | RASQDISSYLA |
| 7 | CDR-L2 | ATSTLQS |
| 8 | CDR-L3 | QQLNSYPWT |
| 9 | Ab heavy chain sequence with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPGK |
| 10 | Ab light chain | DIQLTQSPSFLSASVGDRVTITCRASQDISSYL AWYQQEPGKAPKLLIYATSTLQSGVPSRFSG SGSGTEFTLTITSLQPEDFATYYCQQLNSYPW TFGQGTKVEIKRTVAAPSVFIFPPSDEQLKSG TASVVCLLNNFYPREAKVOWKVDNALOSGN |
| | | SQESVTEQDSKDSTYSLSSTLTLSKADYEKHK VYACEVTHQGLSSPVTKSFNRGEA |
| 11 | Ab heavy chain sequence with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPG |
| 12 | Ab IgG1 heavy chain STR with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPGK |
| 13 | Ab IgG1 heavy chain STR with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS |
| | | KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPG |
| 14 | Ab IgG1 heavy chain LALA with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPGK |
| 15 | Ab IgG1 heavy chain LALA with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPG |
| 16 | Ab IgG1 heavy chain YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLYITREPEVTCVVVDVSHEDPEVKFN |
| | | WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPGK |
| 17 | Ab IgG1 heavy chain YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLYITREPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPG |
| 18 | Ab IgG1 heavy chain STR+YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLYITREPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPGK |
| 19 | Ab IgG1 heavy chain STR+YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK |
| | | KVEPKSCDKTHTCPPCPAPESTRGPSVFLFPP KPKDTLYITREPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPG |
| 20 | Ab IgG1 heavy chain LALA+YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLYITREPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPGK |
| 21 | Ab IgG1 heavy chain LALA+YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLYITREPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHNHYTQKSLSLSPG |
| 22 | Ab IgG1 heavy chain REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP |
| | | EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVLHRDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVEHE ALHWHYTQKSLSLSPGK |
| 23 | Ab IgG1 heavy chain REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVLHRDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVEHE ALHWHYTQKSLSLSPG |
| 24 | Ab IgG1 heavy chain STR+REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVLHRDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVEHE ALHWHYTQKSLSLSPGK |
| 25 | Ab IgG1 heavy chain STR+REW with C-terminal lysine truncation | EVQLVESGGGLVQPGGSLRLSCAASGFTFSG FGMNWVRQAPGKGLEWVSYTSSSSRTIYYA DSVKGRFTISRDNAONSLYLOMNSLRDEDTA |
| | | VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVLHRDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVEHE ALHWHYTQKSLSLSPG |
| 26 | Ab IgG1 heavy chain LALA+REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVLHRDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVEHE ALHWHYTQKSLSLSPGK |
| 27 | Ab IgG1 heavy chain LALA+REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVLHRDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVEHE ALHWHYTQKSLSLSPG |
| 28 | Ab IgG1 heavy chain LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHSHYTQKSLSLSPGK |
| 29 | Ab IgG1 heavy chain LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHSHYTQKSLSLSPG |
| 30 | Ab IgG1 heavy chain STR+LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD |
| | | SDGSFFLYSKLTVDKSRWQQGNVFSCSVLHE ALHSHYTQKSLSLSPGK |
| 31 | Ab IgG1 heavy chain STR+LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHSHYTQKSLSLSPG |
| 32 | Ab IgG1 heavy chain LALA+LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHSHYTQKSLSLSPGK |
| 33 | Ab IgG1 heavy chain LALA+LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVLHQDWLNGKEYKCKVSNKALPAPIEKTIS |
| | | KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHSHYTQKSLSLSPG |
| 34 | Ab IgG1 heavy chain QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDQLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHNHYTQKSLSLSPGK |
| 35 | Ab IgG1 heavy chain QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDQLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHNHYTQKSLSLSPG |
| 36 | Ab IgG1 heavy chain STR+QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDQLMISRTPEVTCVVVDVSHEDPEVKFN |
| | | WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHNHYTQKSLSLSPGK |
| 37 | Ab IgG1 heavy chain STR+QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDQLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHNHYTQKSLSLSPG |
| 38 | Ab IgG1 heavy chain LALA+QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDQLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHNHYTQKSLSLSPGK |
| 39 | Ab IgG1 heavy chain LALA+QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTOTYICNVNHKPSNTKVDK |
| | | KVEPKSCDKTHTCPPCPAPEAAGGPSVFLFPP KPKDQLMISRTPEVTCVVVDVSHEDPEVKFN WYVDGVEVHNAKTKPREEQYNSTYRVVSVL TVLHQDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVLHE ALHNHYTQKSLSLSPG |
| 40 | Ab IgG1 heavy chain DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVDHHDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHSHYTQKSLSLSPGK |
| 41 | Ab IgG1 heavy chain DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPELLGGPSVFLFPP KPKDTLMISRTPEVTCVVVDVSHEDPEVKFN WYVDGVEVHNAKTKPREEQYNSTYRVVSVL TVDHHDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHSHYTQKSLSLSPG |
| 42 | Ab IgG1 heavy chain STR+DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP |
| | | EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVDHHDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHSHYTQKSLSLSPGK |
| 43 | Ab IgG1 heavy chain STR+DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPESTRGPSVFLFPP KPKDTLMISRTPEVTCVVVDVSHEDPEVKFN WYVDGVEVHNAKTKPREEQYNSTYRVVSVL TVDHHDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHSHYTQKSLSLSPG |
| 44 | Ab IgG1 heavy chain LALA+DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVDHHDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHSHYTQKSLSLSPGK |
| 45 | Ab IgG1 heavy chain LALA+DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA |
| | | VYYCARDSSGWYDAFDIWGQGTMVTVSSAS TKGPSVFPLAPSSKSTSGGTAALGCLVKDYFP EPVTVSWNSGALTSGVHTFPAVLQSSGLYSL SSVVTVPSSSLGTQTYICNVNHKPSNTKVDK KVEPKSCDKTHTCPPCPAPEAAGGPSVFLFPP KPKDTLMISRTPEVTCVVVDVSHEDPEVKFN WYVDGVEVHNAKTKPREEQYNSTYRVVSVL TVDHHDWLNGKEYKCKVSNKALPAPIEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMH EALHSHYTQKSLSLSPG |
| 46 | Ab IgG4 S228P heavy chain with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 47 | Ab IgG4 S228P heavy chain with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 48 | Ab IgG4 S228P heavy chain STR with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLGK |
| 49 | Ab IgG4 S228P heavy chain STR with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLG |
| 50 | Ab IgG4 S228P heavy chain FALA with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG |
| | | SFFLYSRLTVDKSRWQEGNVFSCSVMHEALH NHYTQKSLSLSLGK |
| 51 | Ab IgG4 S228P heavy chain FALA with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 52 | Ab IgG4 S228P heavy chain YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 53 | Ab IgG4 S228P heavy chain YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK |
| | | GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 54 | Ab IgG4 S228P heavy chain STR+YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLYITREPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLGK |
| 55 | Ab IgG4 S228P heavy chain STR+YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLYITREPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLG |
| 56 | Ab IgG4 S228P heavy chain FALA+YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLYITREPEVTCVVVDVSQEDPEVQFNWYV |
| | | DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 57 | Ab IgG4 S228P heavy chain FALA+YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 58 | Ab IgG4 S228P heavy chain REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLGK |
| 59 | Ab IgG4 S228P heavy chain REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK |
| | | RVESKYGPPCPPCPAPEFLGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLG |
| 60 | Ab IgG4 S228P heavy chain STR+REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVLHR DWLNGKEYKCKVSNKGLPSSIEKTISKAKGQ PREPQVYTLPPSQEEMTKNQVSLTCLVKGFY PSDIAVEWESNGQPENNYKTTPPVLDSDGSFF LYSRLTVDKSRWQEGNVFSCSVEHEALHWH YTQKSLSLSLGK |
| 61 | Ab IgG4 S228P heavy chain STR+REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVLHR DWLNGKEYKCKVSNKGLPSSIEKTISKAKGQ PREPQVYTLPPSQEEMTKNQVSLTCLVKGFY PSDIAVEWESNGQPENNYKTTPPVLDSDGSFF LYSRLTVDKSRWQEGNVFSCSVEHEALHWH YTQKSLSLSLG |
| 62 | Ab IgG4 S228P heavy chain FALA+REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP |
| | | EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLGK |
| 63 | Ab IgG4 S228P heavy chain FALA+REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLG |
| 64 | Ab IgG4 S228P heavy chain LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLGK |
| 65 | Ab IgG4 S228P heavy chain LS with C-terminal lysine truncation | EVQLVESGGGLVQPGGSLRLSCAASGFTFSG FGMNWVRQAPGKGLEWVSYTSSSSRTIYYA DSVKGRFTISRDNAONSLYLOMNSLRDEDTA |
| | | VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLG |
| 66 | Ab IgG4 S228P heavy chain STR+LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVLHEALHS HYTQKSLSLSLGK |
| 67 | Ab IgG4 S228P heavy chain STR+LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVLHEALHS HYTQKSLSLSLG |
| 68 | Ab IgG4 S228P heavy chain FALA+LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLGK |
| 69 | Ab IgG4 S228P heavy chain FALA+LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLG |
| 70 | Ab IgG4 S228P heavy chain QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD |
| | | GSFFLYSRLTVDKSRWQEGNVFSCSVLHEAL HNHYTQKSLSLSLGK |
| 71 | Ab IgG4 S228P heavy chain QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD GSFFLYSRLTVDKSRWQEGNVFSCSVLHEAL HNHYTQKSLSLSLG |
| 72 | Ab IgG4 S228P heavy chain STR+QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD QLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH NHYTQKSLSLSLGK |
| 73 | Ab IgG4 S228P heavy chain STR+QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD QLMISRTPEVTCVVVDVSQEDPEVQFNWYV DGVEVHNAKTKPREEQFNSTYRVVSVLTVL HQDWLNGKEYKCKVSNKGLPSSIEKTISKAK |
| | | GQPREPQVYTLPPSQEEMTKNQVSLTCLVKG FYPSDIAVEWESNGQPENNYKTTPPVLDSDG SFFLYSRLTVDKSRWQEGNVFSCSVLHEALH NHYTQKSLSLSLG |
| 74 | Ab IgG4 S228P heavy chain FALA+QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD GSFFLYSRLTVDKSRWQEGNVFSCSVLHEAL HNHYTQKSLSLSLGK |
| 75 | Ab IgG4 S228P heavy chain FALA+QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD GSFFLYSRLTVDKSRWQEGNVFSCSVLHEAL HNHYTQKSLSLSLG |
| 76 | Ab IgG4 S228P heavy chain DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV |
| | | DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLGK |
| 77 | Ab IgG4 S228P heavy chain DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEFLGGPSVFLFPPKPK DTLMISRTPEVTCVVVDVSQEDPEVQFNWYV DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLG |
| 78 | Ab IgG4 S228P heavy chain STR+DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPESTRGPSVFLFPPKPKD TLMISRTPEVTCVVVDVSQEDPEVQFNWYVD GVEVHNAKTKPREEQFNSTYRVVSVLTVDH HDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALHS HYTQKSLSLSLGK |
| 79 | Ab IgG4 S228P heavy chain STR+DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK |
| | | RVESKYGPPCPPCPAPESTRGPSVFLFPPKPKD TLMISRTPEVTCVVVDVSQEDPEVQFNWYVD GVEVHNAKTKPREEQFNSTYRVVSVLTVDH HDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALHS HYTQKSLSLSLG |
| 80 | Ab IgG4 S228P heavy chain FALA+DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLGK |
| 81 | Ab IgG4 S228P heavy chain FALA+DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPPCPAPEAAGGPSVFLFPPKPK DTLMISRTPEVTCVVVDVSQEDPEVQFNWYV DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLG |
| 82 | Ab IgG4 heavy chain with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP |
| | | EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 83 | Ab IgG4 heavy chain with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 84 | Ab IgG4 heavy chain STR with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLGK |
| 85 | Ab IgG4 heavy chain STR with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA |
| | | VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLG |
| 86 | Ab IgG4 heavy chain FALA with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 87 | Ab IgG4 heavy chain FALA with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 88 | Ab IgG4 heavy chain YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 89 | Ab IgG4 heavy chain YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 90 | Ab IgG4 heavy chain STR+YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPK[?] TLYITREPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS |
| | | FFLYSRLTVDKSRWQEGNVFSCSVMHEALH NHYTQKSLSLSLGK |
| 91 | Ab IgG4 heavy chain STR+YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLYITREPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALH NHYTQKSLSLSLG |
| 92 | Ab IgG4 heavy chain FALA+YTE with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLGK |
| 93 | Ab IgG4 heavy chain FALA+YTE with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLYITREPEVTCVVVDVSQEDPEVQFNWYV DGVEVHNAKTKPREEQFNSTYRVVSVLTVL HQDWLNGKEYKCKVSNKGLPSSIEKTISKAK |
| | | GQPREPQVYTLPPSQEEMTKNQVSLTCLVKG FYPSDIAVEWESNGQPENNYKTTPPVLDSDG SFFLYSRLTVDKSRWQEGNVFSCSVMHEALH NHYTQKSLSLSLG |
| 94 | Ab IgG4 heavy chain REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLGK |
| 95 | Ab IgG4 heavy chain REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLG |
| 96 | Ab IgG4 heavy chain STR+REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD |
| | | GVEVHNAKTKPREEQFNSTYRVVSVLTVLHR DWLNGKEYKCKVSNKGLPSSIEKTISKAKGQ PREPQVYTLPPSQEEMTKNQVSLTCLVKGFY PSDIAVEWESNGQPENNYKTTPPVLDSDGSFF LYSRLTVDKSRWQEGNVFSCSVEHEALHWH YTQKSLSLSLGK |
| 97 | Ab IgG4 heavy chain STR+REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVLHR DWLNGKEYKCKVSNKGLPSSIEKTISKAKGQ PREPQVYTLPPSQEEMTKNQVSLTCLVKGFY PSDIAVEWESNGQPENNYKTTPPVLDSDGSFF LYSRLTVDKSRWQEGNVFSCSVEHEALHWH YTQKSLSLSLG |
| 98 | Ab IgG4 heavy chain FALA+REW with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLGK |
| 99 | Ab IgG4 heavy chain FALA+REW with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK |
| | | RVESKYGPPCPSCPAPEAAGGPSVFLFPPKPK DTLMISRTPEVTCVVVDVSQEDPEVQFNWYV DGVEVHNAKTKPREEQFNSTYRVVSVLTVL HRDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVEHEALH WHYTQKSLSLSLG |
| 100 | Ab IgG4 heavy chain LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLGK |
| 101 | Ab IgG4 heavy chain LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLG |
| 102 | Ab IgG4 heavy chain STR+LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP |
| | | EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVLHEALHS HYTQKSLSLSLGK |
| 103 | Ab IgG4 heavy chain STR+LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVLH QDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVLHEALHS HYTQKSLSLSLG |
| 104 | Ab IgG4 heavy chain FALA+LS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLGK |
| 105 | Ab IgG4 heavy chain FALA+LS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA |
| | | VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH SHYTQKSLSLSLG |
| 106 | Ab IgG4 heavy chain QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD GSFFLYSRLTVDKSRWQEGNVFSCSVLHEAL HNHYTQKSLSLSLGK |
| 107 | Ab IgG4 heavy chain QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD GSFFLYSRLTVDKSRWQEGNVFSCSVLHEAL HNHYTQKSLSLSLG |
| 108 | Ab IgG4 heavy chain STR+QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD QLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH NHYTQKSLSLSLGK |
| 109 | Ab IgG4 heavy chain STR+QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD QLMISRTPEVTCVVVDVSQEDPEVQFNWYV DGVEVHNAKTKPREEQFNSTYRVVSVLTVL HQDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVLHEALH NHYTQKSLSLSLG |
| 110 | Ab IgG4 heavy chain FALA+QL with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD |
| | | GSFFLYSRLTVDKSRWQEGNVFSCSVLHEAL HNHYTQKSLSLSLGK |
| 111 | Ab IgG4 heavy chain FALA+QL with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DQLMISRTPEVTCVVVDVSQEDPEVQFNWY VDGVEVHNAKTKPREEQFNSTYRVVSVLTV LHQDWLNGKEYKCKVSNKGLPSSIEKTISKA KGQPREPQVYTLPPSQEEMTKNQVSLTCLVK GFYPSDIAVEWESNGQPENNYKTTPPVLDSD GSFFLYSRLTVDKSRWQEGNVFSCSVLHEAL HNHYTQKSLSLSLG |
| 112 | Ab IgG4 heavy chain DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLGK |
| 113 | Ab IgG4 heavy chain DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEFLGGPSVFLFPPKPK DTLMISRTPEVTCVVVDVSQEDPEVQFNWYV DGVEVHNAKTKPREEQFNSTYRVVSVLTVD HHDWLNGKEYKCKVSNKGLPSSIEKTISKAK |
| | | GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLG |
| 114 | Ab IgG4 heavy chain STR+DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVDH HDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALHS HYTQKSLSLSLGK |
| 115 | Ab IgG4 heavy chain STR+DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPESTRGPSVFLFPPKPKD TLMISRTPEVTCVVVDVSQEDPEVQFNWYVD GVEVHNAKTKPREEQFNSTYRVVSVLTVDH HDWLNGKEYKCKVSNKGLPSSIEKTISKAKG QPREPQVYTLPPSQEEMTKNQVSLTCLVKGF YPSDIAVEWESNGQPENNYKTTPPVLDSDGS FFLYSRLTVDKSRWQEGNVFSCSVMHEALHS HYTQKSLSLSLG |
| 116 | Ab IgG4 heavy chain FALA+DHS with C-terminal lysine | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV |
| | | DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLGK |
| 117 | Ab IgG4 heavy chain FALA+DHS with C-terminal lysine truncation | EVQLVESGGGLVQPGGSLRLSCAASGFTFSG FGMNWVRQAPGKGLEWVSYTSSSSRTIYYA DSVKGRFTISRDNAQNSLYLQMNSLRDEDTA VYYCARDSSGWYDAFDIWGQGTMVTVSSAS TKGPSVFPLAPCSRSTSESTAALGCLVKDYFP EPVTVSWNSGALTSGVHTFPAVLQSSGLYSL SSVVTVPSSSLGTKTYTCNVDHKPSNTKVDK RVESKYGPPCPSCPAPEAAGGPSVFLFPPKPK DTLMISRTPEVTCVVVDVSQEDPEVQFNWYV DGVEVHNAKTKPREEQFNSTYRVVSVLTVD HHDWLNGKEYKCKVSNKGLPSSIEKTISKAK GQPREPQVYTLPPSQEEMTKNQVSLTCLVKG FYPSDIAVEWESNGQPENNYKTTPPVLDSDG SFFLYSRLTVDKSRWQEGNVFSCSVMHEALH SHYTQKSLSLSLG |

## Claims

1. An anti-GPC3 antibody drug conjugate of formula (IV): 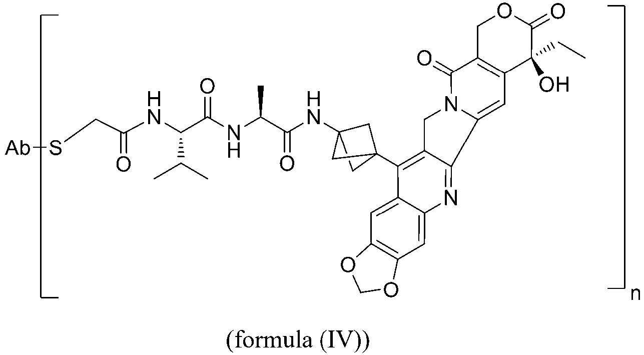 wherein n is an integer from 1 to 6, and wherein Ab is an anti-GPC3 antibody comprising a heavy chain variable region comprising a CDR-H1, a CDR-H2, and a CDR-H3; and a light chain variable region comprising a CDR-L1, a CDR-L2, and a CDR-L3, wherein
CDR-H1 has the amino acid sequence shown as SEQ ID NO: 3,
CDR-H2 has the amino acid sequence shown as SEQ ID NO: 4,
CDR-H3 has the amino acid sequence shown as SEQ ID NO: 5;
CDR-L1 has the amino acid sequence shown as SEQ ID NO: 6,
CDR-L2 has the amino acid sequence shown as SEQ ID NO: 7, and
CDR-L3 has the amino acid sequence shown as SEQ ID NO: 8.

2. The anti-GPC3 antibody drug conjugate according to claim 1, wherein the antibody is an IgG1 antibody.

3. The anti-GPC3 antibody drug conjugate according to claim 1 or 2, wherein the antibody comprises
(a) a heavy chain variable region having the amino acid sequence shown as SEQ ID NO: 1 and
(b) a light chain variable region having the amino acid sequence shown as SEQ ID NO: 2.

4. The anti-GPC3 antibody drug conjugate according to claim 1 or 2, wherein the antibody comprises
(a) a heavy chain having the amino acid shown as SEQ ID NO: 9 or SEQ ID NO: 11 and
(b) a light chain having the amino acid sequence shown as SEQ ID NO: 10.

5. An anti-GPC3 antibody drug conjugate of formula (IV): 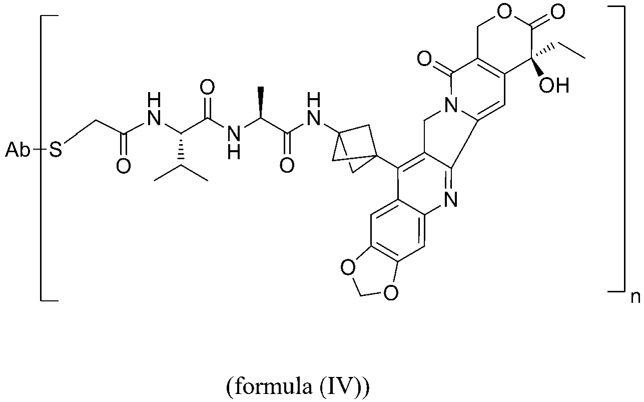 wherein n is an integer from 1 to 6, and wherein Ab is an anti-GPC3 antibody comprising: two heavy chains each having an amino acid sequence shown as SEQ ID NO: 9 and two light chains each having an amino acid sequence shown as SEQ ID NO: 10.

6. The anti-GPC3 antibody drug conjugate according to any one of claims 1-5, wherein n is 2.

7. The anti-GPC3 antibody drug conjugate according to any one of claims 1-5, wherein n is 1.

8. The anti-GPC3 antibody drug conjugate according to any one of claims 1-5, wherein n is 4.

9. The anti-GPC3 antibody drug conjugate according to any one of claims 1-5, wherein n is 6.

10. A composition comprising a therapeutically effective amount of an antibody drug conjugate according to any of claims 1-5, wherein the predominant species of the antibody drug conjugate in the composition has an n of 2.

11. A pharmaceutical composition comprising a therapeutically effective amount of an antibody drug conjugate according to any of claims 1-5 and a pharmaceutically acceptable excipient, wherein the composition has a DAR of 2 or about 2.

12. An antibody drug conjugate according to any of claims 1-9 for use in the treatment of hepatocellular carcinoma.

13. A pharmaceutical composition according to claim 11 for use in the treatment of hepatocellular carcinoma.

14. One or more nucleic acids encoding an antibody comprising a heavy chain sequence of SEQ ID NO: 9 or SEQ ID NO: 11, and a light chain sequence of SEQ ID NO: 10.
